# EUROPEAN PATENT SPECIFICATION

(11) **EP 4 237 022 B1**
(45) Date of publication and mention of the grant of the patent: **26.08.2026**
(21) Application number: 21811615.0
(22) Date of filing: 27.10.2021
(51) Int. Cl.: A61L 27/20, A61L 27/50, A61L 27/52, A61Q 19/00

(54) **TRANSDERMAL DELIVERY**
TRANSDERMALE VERABREICHUNG
ADMINISTRATION TRANSDERMIQUE

(30) Priority: 28.10.2020 US 202063106623 P
(43) Date of publication of application: 06.09.2023
(73) Proprietor: Fount Bio, Inc., Cambridge, MA 02139 (US)
(72) Inventor: LEVINSON, Douglas, Sherborn, Massachusetts 01770 (US); VOGUS, Douglas R., Cambridge, Massachusetts 02140 (US); KRISHNAN, Vinu, Brookline, Massachusetts 02446 (US); MITRAGOTRI, Samir, Lexington, Massachusetts 02420 (US)
(74) Representative: EIP
(86) International application number: PCT/US2021/056916
(87) International publication number: WO 2022/094002

(56) References cited:
- CA-A1- 3 118 491
- US-A1- 2004 127 699
- US-A1- 2014 227 174
- US-B2- 10 624 865

## Description

### Priority Claim

The benefit of priority is claimed to United States Provisional Application Number 63/106,623, filed 28 October 2020 (28.10.2020).

### Background

Various systems have been developed for the installation or generation of crosslinked materials at or on a particular site of interest, for example at a site of surgical or traumatic disruption (*e.g*., of organs, connective tissues, muscles, tendons and/or membranes; see, for example, Seal et al. Mater Sci. Eng 34:147, 2001). Some such materials have shown promise, for example, in effectively sealing internal wounds and/or achieving tissue approximation for improved wound healing (see, for example, Ruel-Gariepy et al. Eur. J. Pharm. Biopharm. 58:409, 2004). CA 3 118 491 A1 discloses a cross-linkable composition for application into skin using microneedles. In an embodiment the composition comprises hyaluronic acid functionalized with CBT and Cys-PEG-Cys.

### Summary

The present disclosure provides a variety of insights relating to technologies for transdermal delivery of agents (*e.g*., comprising crosslinkable entities), and/or for achieving cosmetic or therapeutic activity or benefit in the skin.

The present disclosure provides an insight that certain advantages may be achieved when microneedling technologies are utilized. For instance, in some embodiments, microneedling technologies may enhance or achieve delivery of agents, e.g., to a particular target site. In some embodiments, microneedling as described herein may improve extent of delivery (*e.g.,* total amount delivered, amount delivered within a unit time, and/or degree - *e.g.,* depth - of penetration).

In some embodiments, the present disclosure provides an insight that a desired rate and/or extent of penetration and/or *in situ* formation of agents can be achieved by controlling one or more particular parameters. Among other things, the present disclosure demonstrates specific embodiments of, one or more parameters related to preparations (*e.g*., identity of agent, and/or one or more characteristics such as molecular weight, lipophilicity, density of functional moieties, viscosity, pH, *etc.),* microneedling *(e.g.,* dimensions [*e.g.,* diameter and/or length] of needles, needle density, *etc*.) , and/or administration methods *(e.g.,* location and/or timing of microneedling relative to application of agent(s), mode and/or timing of administration of agents relative to one another and/or to microneedling) that achieve beneficial results with respect to delivery of agent(s) *(e.g.,* of crosslinkable entities) and/or *in situ* formation of crosslinked material(s).

In various embodiments, provided technologies achieve formation and retention of desirable crosslinked material(s) at intradermal target site(s). Among other things, the present disclosure defines particularly useful sizes and/or concentrations of crosslinkable entities (and/or moieties), molar ratios, conditions (*e.g*., pH, formulation additives, timing) of combination, characteristics of microneedles, and combinations thereof, that achieve transdermal delivery of crosslinkable entities and/or *in situ* production of crosslinked materials, including large and/or complex such crosslinked materials.

Among the particular teachings provided herein is that low pH *(e.g.,* lower than physiological pH), in some embodiments, may contribute to increased production of a crosslinked material *(e.g.,* a large and/or complex crosslinked material) *in situ* after topical application of crosslinkable entity(ies) as described herein. Without wishing to be bound by any particular theory, the present disclosure proposes that such low pH may slow reaction time between crosslink moieties, and/or may otherwise permit penetration before crosslinking is complete, so that crosslinkable entity(ies) can penetrate before crosslinking is complete (and thus before a crosslinked material that is too large to penetrate is formed).

The invention provides a method of establishing a crosslinked material at an intradermal target site, the method comprising steps of: (i) applying first and second crosslinkable entities to a skin location at a pH within a range of 3 ± 1% to 5 ± 1%; and (ii) microneedling the skin location, such that the crosslinked material becomes present at an intradermal target site, wherein: (a) the first crosslinkable entity comprises a crosslink moiety which is or comprises cyanobenzothiazole (CBT), a CBT mimetic, or another molecule that reacts with either the -SH or the NH2 group of cysteine; and (b) the second crosslinkable entity comprises a crosslink moiety which is or comprises cysteine (Cys), e.g., D-Cys, L-Cys, or combinations thereof.

In some embodiments, the presence of the crosslinked material is determinable at the intradermal target site. In some embodiments, the crosslinked material becomes present within a time period of 1 minute to 1 hour.

In some embodiments, the microneedling is performed after the applying step. In some embodiments, the microneedling is performed before the applying step. In some embodiments, the microneedling is performed before and after the applying step.

In some embodiments, the first crosslinkable entity comprises a polymer moiety and a weight averaged molecular weight of the polymer moiety prior to the applying step is within a range of 1kDa to 500kDa. In some embodiments, the weight averaged molecular weight of the polymer moiety prior to the applying step is within a range of 5 to 20 kDa.

In some embodiments, the polymer moiety is hyaluronic acid ("HA") polymer.

In some embodiments, the method further comprises applying to the skin location the first and second crosslinkable entities.

In some embodiments, the first and second crosslinkable entities are applied simultaneously. In some embodiments, the method further comprises mixing the first and second crosslinkable entities before the applying step. In some embodiments, the mixing step is performed 0 to 30 minutes before the applying step.

In some embodiments, the second crosslinkable entity is applied after the first crosslinkable entity.

In some embodiments, the microneedling is performed with a microneedle device having microneedles. In some embodiments, the microneedle device has a microneedle density within a range of about 20-150 microneedles/cm². In some embodiments, the microneedle device has 1 to 100000 microneedles. In some embodiments, the microneedles have a length between about 100 µm and about 1000 µm. In some embodiments, the microneedle device is a dermaroller.

In some embodiments, the intradermal site is epidermis (e.g., stratum corneum, stratum lucidum, stratum granulosum, stratum spinosum, stratum basale), or dermis.

In some embodiments, a concentration of the first crosslinkable entity is within a range of 0.1 to 100 mg/mL.

In some embodiments, the second crosslinkable entity is selected from the group consisting of Cysteine-Ethylenediamine-Cysteine (CEC), Cysteine-Lysine-Cysteine (CKC), Cysteine-PEG-Cysteine, and combinations thereof.

In some embodiments, a concentration of the second crosslinkable entity is within a range of 0.1 to 100 mg/mL.

In some embodiments, a molecular ratio of the first crosslink moiety and the second crosslink moiety is within a range of 1:1 to 5:1.

In some embodiments, the first crosslinkable entity comprises a first crosslink moiety, and 1-20 mol % of the first crosslinkable entity comprises the first crosslink moiety.

In some embodiments, the crosslinked material is characterized that a weight averaged molecular weight of the crosslinked material at the intradermal target site is *(e.g.,* two, three, four, five, six, seven, eight, nine, ten times) greater than a weight averaged molecular weight of the first crosslinkable entity.

### Brief Description of the Drawing

FIGS. 1A and 1B depict linear hyaluronic acid-6-amino-2-cyanobenzothiazole (HA-CBT) concentrations delivered when HA-CBT was administered to a tape-stripped skin and administered via microneedling. FIG. 1A shows the result from HA-CBT having a HA molecular weight of 10 kDa (HA₁₀CBT). FIG. 1B shows the result from HA-CBT having a HA molecular weight of 20 kDa (HA₂₀CBT).
FIG. 2 shows HA-CBT concentrations in that epidermis with the stratum corneum and dermis when HA-CBT was administered via microneedling.
FIG. 3 depicts HA-CBT concentrations in the upper stratum corneum, lower stratum corneum, epidermis, and dermis. HA₁₀CBT was administered before or after microneedling (e.g., rolling dermaroller).
FIG. 4 shows HA-CBT concentrations in the epidermis including stratum corneum and dermis. HA₁₀CBT was administered in combination with microneedling and incubated at 37 °C for 1 hour or overnight.
FIGS. 5A-5C are gel permeation chromatography (GPC) chromatographs of HA₁₀CBT after crosslinking with CKC at different concentrations. For FIGS. 5A and 5B, crosslinked structures were formed from mixing different concentrations of HA₁₀CBT with a stoichiometric equivalent of CKC. For FIG. 5C, crosslinked structures were formed by combining a constant concentration of HA₁₀CBT (1 mg/mL) with different molar ratios (R) of CKC.
FIGS. 6A and 6B are GPC chromatographs of HA₁₀CBT after crosslinking with CKC in the presence of human skin homogenate. HA₁₀CBT (1 mg/mL) was incubated with or without skin (±skin) and then mixed with CKC (±XL) at defined time points (0, 30, and 90 min.) for FIG. 6A. HA₁₀CBT (2 mg/mL) was incubated with skin (+skin) and then mixed with CKC (±XL) at defined time points (0 and 90 min.) for FIG. 6B.
FIG. 7 is a GPC chromatograph of HA₁₀CBT with or without the crosslinker cysteine-ethylenediamine-cysteine (CEC) in phosphate-buffered saline PBS (pH=7.4) and citrate buffer (25 mM, pH=4.6). HA₁₀CBT (20 mg/mL) was mixed with CEC (equimolar CBT:Cys concentration) and the crosslinking reaction was quenched at each defined time point by diluting the polymer concentration to 1 mg/mL and then run on GPC.

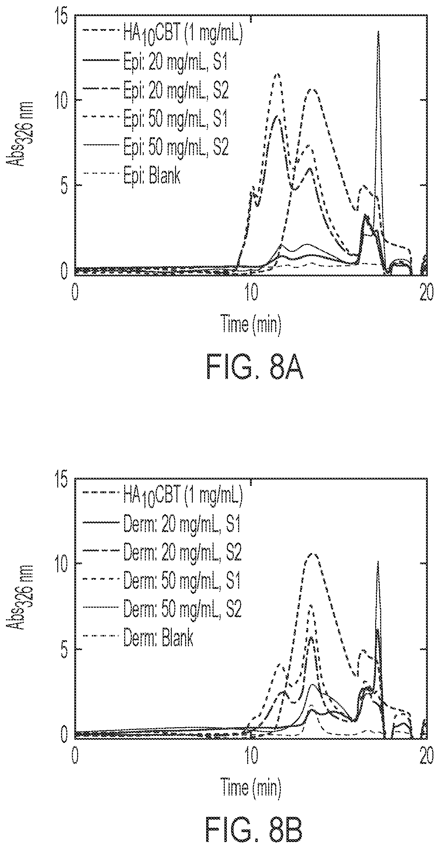

FIGS. 8A and 8B are GPC chromatographs of epidermal (FIG. 8A) and dermal (FIG. 8B) skin extracts after microneedling HA₁₀CBT and CEC (equimolar CBT:Cys concentration) at different polymer concentrations: 20 mg/mL and 50 mg/mL. Each formulation was applied in acidic buffer: 25 mM citrate, pH=4.6, 0.2% ethylenediaminetetraacetic acid (EDTA).
FIGS. 9A and 9B are GPC chromatographs of epidermal (FIG. 9A) and dermal (FIG. 9B) skin extracts after microneedling HA₁₀CBT (50 mg/ml) and CEC (equimolar CBT:Cys concentration) into the skin. HA₁₀CBT and CEC were mixed immediately prior to topical application in acidic buffer (25 mM citrate, pH = 4.6, 0.2 wt% EDTA) for S1 and S2. The two molecules were mixed 1h prior in acidic buffer (25 mM citrate, pH = 4.6, 0.2 wt% EDTA) for S3 and S4, and in neutral buffer (PBS) for S5 and S6.
FIGS. 10A and 10B are GPC chromatographs of epidermal (FIG. 10A) and dermal (FIG. 10B) skin extracts after microneedling HA₁₀CBT (50 mg/ml) and CEC (equimolar CBT:Cys concentration) into the skin with different microneedle sizes (250, 500, and 1000 µm).
FIGS. 11A and 11B are GPC chromatographs after injection of HA₁₀CBT and CEC into skin simultaneously (FIG. 11A) and sequentially (FIG. 11B).
FIG. 12 shows HA-CBT concentrations in the upper SC, lower SC, epidermis, and dermis when it was administered via microneedling at concentrations of 10 mg/mL and 50 mg/mL in PBS.
FIG. 13 shows HA-CBT concentrations in the upper SC, lower SC, epidermis, and dermis when it was administered with 250 µm and 500 µm dermarollers.
FIGS. 14A-14C are microscopic images that visualize the difference in topical delivery of HA-CBT and CEC using dermarollers of different needle lengths.
FIGS. 15A and 15B are GPC chromatographs that depict, 11 days following application, the detection of HA-CBT in skin extract when HA-CBT is applied without CEC using a dermaroller (FIG. 15A) and the detection of high molecular weight material in skin extract when HA-CBT is applied with CEC using a dermaroller and through injection (FIG. 15B).
FIGS. 16A-16C show the H & E stains for excised pig skin 11 days after (FIG. 16A) application of buffer with a dermaroller, (FIG. 16B) intradermal injection of HA-CBT and CEC, and (FIG. 16C) application of HA-CBT and CEC with a dermaroller.
FIG. 17 shows an H & E stain for pig skin which was excised on day 28 following the application of HA-CBT and CEC with 500 µm dermarollers on day 0 and day 14. HA (shown in blue and pointed by arrows) is observed in the superficial dermis.

### Definitions

***About:*** The term "about", when used herein in reference to a value, refers to a value that is similar, in context to the referenced value. In general, those skilled in the art, familiar with the context, will appreciate the relevant degree of variance encompassed by "about" in that context. For example, in some embodiments, the term "about" may encompass a range of values that are within 25%, 20%, 19%, 18%, 17%, 16%, 15%, 14%, 13%, 12%, 11%, 10%, 9%, 8%, 7%, 6%, 5%, 4%, 3%, 2%, 1%, or less of the referred value.

***Administration:*** As used herein, the term "administration" typically refers to the administration of a composition to a subject or system to achieve delivery of an agent that is, or is included in, the composition. Those of ordinary skill in the art will be aware of a variety of routes that may, in appropriate circumstances, be utilized for administration to a subject, for example a human. For example, in some embodiments, administration may be ocular, oral, parenteral, topical, *etc.* In some particular embodiments, administration may be bronchial *(e.g.,* by bronchial instillation), buccal, dermal (which may be or comprise, for example, one or more of topical to the dermis, intradermal, interdermal, transdermal, *etc*), enteral, intra-arterial, intradermal, intragastric, intramedullary, intramuscular, intranasal, intraperitoneal, intrathecal, intravenous, intraventricular, within a specific organ (e.g. intrahepatic), mucosal, nasal, oral, rectal, subcutaneous, sublingual, topical, tracheal (e.g., by intratracheal instillation), vaginal, vitreal, *etc.* In some embodiments, administration may involve only a single dose. In some embodiments, administration may involve application of a fixed number of doses. In some embodiments, administration may involve dosing that is intermittent (*e.g*., a plurality of doses separated in time) and/or periodic (*e.g*., individual doses separated by a common period of time) dosing. In some embodiments, administration may involve continuous dosing (*e.g*., perfusion) for at least a selected period of time.

***Analog:*** As used herein, the term "analog" refers to a substance that shares one or more particular structural features, elements, components, or moieties with a reference substance. Typically, an "analog" shows significant structural similarity with the reference substance, for example sharing a core or consensus structure, but also differs in certain discrete ways. In some embodiments, an analog is a substance that can be generated from the reference substance, *e.g*., by chemical manipulation of the reference substance. In some embodiments, an analog is a substance that can be generated through performance of a synthetic process substantially similar to (*e.g*., sharing a plurality of steps with) one that generates the reference substance. In some embodiments, an analog is or can be generated through performance of a synthetic process different from that used to generate the reference substance.

***Agent** :* In general, the term "agent", as used herein, may be used to refer to a compound or entity of any chemical class including, for example, a polypeptide, nucleic acid, saccharide, lipid, small molecule, metal, or combination or complex thereof. In appropriate circumstances, as will be clear from context to those skilled in the art, the term may be utilized to refer to an entity that is or comprises a cell or organism, or a fraction, extract, or component thereof. Alternatively or additionally, as context will make clear, the term may be used to refer to a natural product in that it is found in and/or is obtained from nature. In some instances, again as will be clear from context, the term may be used to refer to one or more entities that is man-made in that it is designed, engineered, and/or produced through action of the hand of man and/or is not found in nature. In some embodiments, an agent may be utilized in isolated or pure form; in some embodiments, an agent may be utilized in crude form. In some embodiments, potential agents may be provided as collections or libraries, for example that may be screened to identify or characterize active agents within them. In some cases, the term "agent" may refer to a compound or entity that is or comprises a polymer; in some cases, the term may refer to a compound or entity that comprises one or more polymeric moieties. In some embodiments, the term "agent" may refer to a compound or entity that is not a polymer and/or is substantially free of any polymer and/or of one or more particular polymeric moieties. In some embodiments, the term may refer to a compound or entity that lacks or is substantially free of any polymeric moiety.

***Associated:*** Two events or entities are "associated" with one another, as that term is used herein, if the presence, level and/or form of one is correlated with that of the other. For example, a particular entity *(e.g.,* polypeptide, genetic signature, metabolite, microbe, *etc.)* is considered to be associated with a particular disease, disorder, or condition, if its presence, level and/or form correlates with incidence of and/or susceptibility to the disease, disorder, or condition (*e.g*., across a relevant population). In some embodiments, two or more entities are physically "associated" with one another if they interact, directly or indirectly, so that they are and/or remain in physical proximity with one another. In some embodiments, two or more entities that are physically associated with one another are covalently linked to one another; in some embodiments, two or more entities that are physically associated with one another are not covalently linked to one another but are non-covalently associated, for example by means of hydrogen bonds, van der Waals interaction, hydrophobic interactions, magnetism, and combinations thereof.

***Biocompatible:*** The term "biocompatible", as used herein, refers to materials that do not cause significant harm to living tissue when placed in contact with such tissue, *e.g., in vivo.* In certain embodiments, materials are "biocompatible" if they are not toxic to cells. In certain embodiments, materials are "biocompatible" if their addition to cells *in vitro* results in less than or equal to 20% cell death, and/or their administration *in vivo* does not induce significant inflammation or other such adverse effects.

***Designed:*** As used herein, the term "designed" refers to an agent (i) whose structure is or was selected by the hand of man; (ii) that is produced by a process requiring the hand of man; and/or (iii) that is distinct from natural substances and other known agents.

***In situ:*** The term "in vitro" as used herein refers to events that occur within a tissue or tissue layer at least one cell layer below the surface of such tissue or tissue layer.

***In vitro:*** The term "in vitro" as used herein refers to events that occur in an artificial environment, e.g., in a test tube or reaction vessel, in cell culture, etc., rather than within a multi-cellular organism.

***In vivo:*** as used herein refers to events that occur within a multi-cellular organism, such as a human and a non-human animal. In the context of cell-based systems, the term may be used to refer to events that occur within a living cell (as opposed to, for example, *in vitro* systems).

***"Improve," "increase", "inhibit"*** or ***"reduce":*** As used herein, the terms "improve", "increase", "inhibit', "reduce", or grammatical equivalents thereof, indicate values that are relative to a baseline or other reference measurement. In some embodiments, an appropriate reference measurement may be or comprise a measurement in a particular system *(e.g.,* in a single individual) under otherwise comparable conditions absent presence of *(e.g.,* prior to and/or after) a particular agent or treatment, or in presence of an appropriate comparable reference agent. In some embodiments, an appropriate reference measurement may be or comprise a measurement in comparable system known or expected to respond in a particular way, in presence of the relevant agent or treatment.

***Physiological conditions:*** as used herein, has its art-understood meaning referencing conditions under which cells or organisms live and/or reproduce. In some embodiments, the term refers to conditions of the external or internal milieu that may occur in nature for an organism or cell system. In some embodiments, physiological conditions are those conditions present within the body of a human or non-human animal, especially those conditions present at and/or within a target site of interest. Physiological conditions typically include one or more of, *e.g.,* a temperature within the range of 20 - 40 °C (and specifically about 37 °C), atmospheric pressure of 1, pH of 6-8, glucose concentration of 1-20 mM, oxygen concentration at atmospheric levels, and gravity as it is encountered on earth.

***Subject:*** As used herein, the term ***"subject"*** refers to any organism to which a provided system is or may be administered, *e.g*., for experimental, diagnostic, prophylactic, cosmetic, and/or therapeutic purposes. Typical subjects include animals (*e.g*., mammals such as mice, rats, rabbits, non-human primates, and/or humans). In some embodiments, a subject is a human. In some embodiments, a subject is suffering from or susceptible to one or more disorders or conditions. In some embodiments, a subject displays one or more symptoms of a disorder or condition. In some embodiments, a subject has been diagnosed with one or more disorders or conditions. In some embodiments, the disorder or condition is or includes cancer, or presence of one or more tumors. In some embodiments, the subject is receiving or has received certain therapy to diagnose and/or to treat a disease, disorder, or condition. In some embodiments, a subject refers to a human seeking cosmetic benefit and/or improvement, such as an improvement of appearance and/or feel of skin.

***Therapeutic agent:*** As used herein, the phrase "therapeutic agent" in general refers to any agent that elicits a desired pharmacological effect (which may, in some embodiments, be or comprise a cosmetic effect) when administered to an organism. In some embodiments, an agent is considered to exhibit an effect (i.e., to be a therapeutic agent) if it demonstrates a statistically significant effect across an appropriate population. In some embodiments, the appropriate population may be a population of model organisms. In some embodiments, an appropriate population may be defined by particular criteria, such as a certain age group, gender, genetic background, preexisting clinical conditions, *etc.,* or combinations thereof. In some embodiments, a therapeutic agent is a substance that can be used to alleviate, ameliorate, relieve, inhibit, prevent, delay onset of, reduce severity of, and/or reduce incidence of one or more symptoms or features of a disease, disorder, and/or condition. In some embodiments, a therapeutic agent is one that achieves a cosmetic effect (i.e., is a cosmetic agent). In some embodiments, a therapeutic agent can be used to achieve improvement of appearance and/or feel of skin, and/or another cosmetic benefit.

***Treat:*** As used herein, the term "treat," "treatment," or "treating" refers to partial or complete alleviation, amelioration, delay of onset of, inhibition, prevention, relief, and/or reduction in incidence and/or severity of one or more symptoms or features of a disease, disorder, and/or condition, or achievement of another desired physiological effect (*e.g.,* a desired cosmetic effect such as improvement of appearance and/or feel of skin, such as visible and/or tactile improvement to skin. In some embodiments, treatment comprises administration of an agent which results in a physiological effect. In some embodiments treatment comprises a cosmetic treatment which upon administration improves physical appearance in manner described herein. In some embodiments, treatment may be administered to a subject who does not exhibit signs or features of a disease, disorder, and/or condition (*e.g*., may be prophylactic). In some embodiments, treatment may be administered to a subject who exhibits only early or mild signs or features of the disease, disorder, and/or condition, for example for the purpose of decreasing the risk of developing pathology associated with the disease, disorder, and/or condition. In some embodiments, treatment may be administered to a subject who exhibits established, severe, and/or late-stage signs of the disease, disorder, or condition.

### Detailed Description of Certain Embodiments

The following description is for the illustration and exemplification of the present disclosure only and is not intended to limit the present disclosure to the specific embodiments described herein. Unless defined otherwise, technical and scientific terms have the same meaning as commonly understood by one of ordinary skill in the art to which this disclosure belongs. All references cited herein, including patent applications and publications, are incorporated by reference in their entirety.

Human skin is multi-layered, comprising an external epidermis, a dermis, and an underlying hypodermis. The epidermis has several layers of tissue, namely, stratum corneum, stratum lucidum, stratum granulosum, stratum spinosum, and stratum basale (identified in order from the outer surface of the skin inward). The skin acts as a barrier to separate and protect the body from its environment. The uppermost layer of the skin, the stratum corneum, imposes limitations for successful delivery of agents due to its formidable barrier properties. The stratum corneum is typically about 10-15 µm thick, and it consists of flattened, keratinized cells (corneocytes) arranged in several layers. The rest of the epidermis below the stratum corneum is approximately 150 µm thick. The dermis is about 1-2 mm thick and is located below the epidermis. The dermis is innervated by various capillaries as well as neuronal processes.

It is understood in the art that human skin restricts transport of compounds with a molecular weight *(e.g.,* a number or weight average molecular weight) above about 500 Daltons (Bos and Meinardi, Experimental Dermatology, 9:165, 2000), and even for such small compounds, effective transport often requires additional manipulation, such as administration of a chemical or physical abrading or disrupting agent and/or of electrical current or magnetic field, *etc.*

In accordance with one or more embodiments, the present disclosure provides certain technologies relating to penetration of agents at a target site which, for example, may be a site in or on skin, for example on, at, in, or below epidermis (e.g., stratum corneum), dermis or underlying hypodermis. Provided technologies achieve desired transporting of an agent across the skin's surface and/or into a target site, for example, desired penetration depth/amount, skin area per administration, reduced pain, recovery time, procedure duration, *etc.* In some embodiments, an agent may be administered in combination with microneedling.

In some embodiments, certain aspects of provided technologies make them particularly useful and/or effective for an agent comprising one or more crosslinkable entities, or capable of forming an *in situ* crosslinked material. In some embodiments, the present disclosure provides technologies for applying first and second crosslinkable entities, selected and/or designed to achieve formation of an *in situ* crosslinked material. Those skilled in the art will appreciate that the formation of this crosslinked material can induce a change in physical properties, such as rheology, and/or a change in the chemical properties, such as an increase in molecular weight. Those skilled in the art will appreciate that these physiochemical changes can produce advantageous benefits for the crosslinked material, compared to the initial crosslinkable entities, such as improved durability/persistence in biological systems.

### Crosslinkable Entities

The present disclosure provides certain technologies relating to administration of crosslinkable entities to a subject, and particular to *in situ* crosslinking of such crosslinkable entities at, in, or on a target site which, for example, may be a site in or on skin, for example on, at, in, or below the epidermis, dermis or underlying hypodermis.

**In** some embodiments, the present disclosure provides technologies for administering a system that comprises first and second crosslinkable entities, selected and/or designed to achieve formation of an *in situ* crosslinked material. Those skilled in the art will appreciate that the formation of this crosslinked material can induce a change in physical properties, such as rheology, and/or a change in the chemical properties, such as an increase in molecular weight. Those skilled in the art will appreciate that these physiochemical changes can produce advantageous benefits for the crosslinked material, compared to the initial crosslinkable entities, such as improved durability/persistence in biological systems.

As described herein, such first and second crosslinkable entities are characterized by an ability, when contacted with one another, to react with one another to form the crosslinked material *in situ, e.g.,* absent administration of a catalyst or other non-participating agent.

In many embodiments, at least one of the crosslinkable entities will comprise a polymer moiety linked with a crosslink moiety. In some embodiments, at least one of the crosslinkable entities will not comprise a polymer moiety. In some embodiments, a pair of crosslinkable entities that react with one another (e.g., absent administration of a catalyst or other non-participating agent) each comprise a polymer moiety linked with a crosslink moiety. In some embodiments, a pair crosslinkable entities that react with one another (e.g., absent administration of a catalyst or other non-participating agent) comprises a first crosslinkable entity that comprises a polymer moiety linked with a crosslink moiety and a second crosslinkable entity that does not comprise a polymer moiety.

In some embodiments, a crosslinkable entity comprises a polymer moiety and a plurality of crosslink moieties, which may be the same or different. Among other things, the present disclosure provides insights and technologies relevant to achieving penetration of crosslinkable entities to a target site in skin *(e.g.,* on, at, in, or below the epidermis, dermis or underlying hypodermis). For example, in some embodiments, the present disclosure teaches that microneedling may permit desirable (e.g., enhanced) penetration of a crosslinkable entity, and particularly of a crosslinkable entity comprising a polymer moiety and a crosslinkable moiety.

Among other things, the present disclosure provides a teaching that microneedling can improve the delivery of crosslinkable entities prior to their crosslinking reaction in skin. The improved delivery of such crosslinkable entities can be designed and/or prepared by modulating lipophilicity and/or molecular weight. For example, in some embodiments, the present disclosure teaches that rate and/or extent of skin penetration by a particular agent (and specifically by an agent that is or comprises a polymer moiety and/or otherwise has a weight averaged molecular weight above 500 daltons, and even within a range of 1-500 kDa) can be enhanced by decreasing the molecular weight of the agent or by increasing lipophilicity of the agent, for example by attaching one or more hydrophobic moieties to the agent.

Alternatively or additionally, in some embodiments, the present disclosure provides technologies relating to providing a crosslinked material in a target site in or on a tissue (and particularly in or on skin), so that contacts between the material and surfaces of the target site are maximized. In some embodiments, such surfaces may include one or more cavities or irregularities, which may, in some embodiments, be micro- or even nano-scale structures. In some embodiments, the present disclosure provides preparations of crosslinkable moieties that have flow characteristics that facilitate such contacts.

In some particular embodiments, a crosslinkable entity for use in accordance with the present disclosure comprises a polymer moiety linked with a crosslink moiety, where the crosslink moiety imparts increased lipophilicity to the conjugate (i.e., to the crosslinkable entity) as compared with the polymer moiety alone; as described herein, in some embodiments, such a crosslink moiety can also improve skin penetration by the crosslinkable entity as compared with that of the polymer moiety alone.

In some embodiments, depth of penetration of a crosslinkable entity will be assessed and/or described in terms of the absolute distance (e.g., in microns) below the surface of the skin. In some embodiments, depth of penetration of a crosslinkable entity will be assessed and/or described in terms of number of cells below the surface of the skin.

In some embodiments, a crosslinkable entity as described herein can and/or does (e.g., when administered as described herein) penetrate to a specified depth into the skin, for example within a particular time period. In some embodiments, a such a specified depth may be, for example, at least 50 microns, at least 100 microns, at least 200 microns, or more; alternatively or additionally, in some embodiments, such specified depth may be at least 2 cell layers, at least 3 cell layers, at least 4 cell layers, at least 5 cell layers, at least 6 cell layers, at least 7 cell layers, at least 8 cell layers, at least 9 cell layers, at least 10 cell layers or more, and/or such time period may be, for example, within 1 day, within 18 hours, within 12 hours, within 11 hours, within 10 hours, within 9 hours, within 8 hours, within 7 hours, within 6 hours, within 5 hours, within 4 hours, within 3 hours, within 2 hours, within 1 hour or less.

In some embodiments, at least 1% of a crosslinkable entity administered to a skin surface penetrates to a target site on, at, in, or below the epidermis, dermis or underlying hypodermis, for example within a time period of 1 day. In some embodiments, skin penetration characteristic(s) of a crosslinkable entity as described herein are observed in the absence of any chemical or physical abrading or disrupting agent and/or of electrical current or magnetic field, *etc (e.g.,* absent a penetration enhancer as understood in the art).

In some embodiments, a crosslinkable entity is characterized by lipophilicity (log P) within a range of about -4 and about 2. In some embodiments, crosslinkable entities described herein comprise a crosslinkable moiety, which is lipophilic. In some embodiments, lipophilicity of a crosslinkable moiety may be determined independently from that of a polymer moiety, and/or of a crosslinkable entity comprising the polymer moiety and one or more crosslinkable moieties. In some embodiments, a crosslinkable moiety is characertized by lipophilicity (log P) within a range of about 0 and about 6. For example, in some embodiments, lipophilicity of a crosslinkable moiety is tested through examination of the crosslinkable moiety prior to association and/or linking with molecule polymer moiety to form a crosslinkable entity.

In some embodiments, lipophilicity for a particular entity or moiety is determined by its partition co-efficient (P) relative to a standard solvent (*e.g*. octanol) and water or solution thereof: $log P = log \frac{\left[Entity or Moiety being assessed in Octanol\right]}{\left[Crosslinkable Entity or Moiety being assessedin PBS\right]}$

In some embodiments, log P of an entity or moiety *(e.g.,* of a crosslinkable entity and/or of a crosslink moiety) useful in accordance with the present disclosure is greater than 0.

In some embodiments, a crosslinkable entity *(e.g.,* a crosslinkable entity with a lipophilicity as described herein) has a molecular weight within a range of 1-1000 kDa. In some embodiments, a system comprises a crosslinkable entity that is about 10-250 kDa. In some embodiments, a system comprises a crosslinkable entity that has a molecular weight *(e.g.,* a weight average molecular weight) within a range of about 10-150 kDa. In some embodiments, a system comprises a crosslinkable entity that has a molecular weight *(e.g.,* a weight average molecular weight) within a range of about 1-500 kDa. In some embodiments, a system comprises a crosslinkable entity that has a molecular weight *(e.g.,* a weight average molecular weight) within a range of about 10-40 kDa. In some embodiments, a system comprises a crosslinkable entity that has a molecular *(e.g.,* a weight average molecular weight) weight of about 10 kDa. In some embodiments, a system comprises a crosslinkable entity that has a molecular weight *(e.g.,* a weight average molecular weight) of about 20 kDa. In some embodiments, a system comprises a crosslinkable entity that has a molecular weight *(e.g.,* a weight average molecular weight) of about 30 kDa. In some embodiments, a system comprises a crosslinkable entity that has a molecular weight *(e.g.,* a weight average molecular weight) of about 40 kDa. In some embodiments, a system comprises a crosslinkable entity that has a molecular weight *(e.g.,* a weight average molecular weight) of about 50 kDa. In some embodiments, a system comprises a crosslinkable entity that has a molecular weight *(e.g.,* a weight average molecular weight) of less than 10 kDa. In some embodiments, a system comprises a crosslinkable entity that has a molecular weight *(e.g.,* a weight average molecular weight) of less than 100 kDa. In some embodiments, a system comprises a crosslinkable entity that has a molecular weight *(e.g.,* a weight average molecular weight) of less than 250 kDa. In some embodiments, a system comprises a crosslinkable entity that has a molecular weight *(e.g.,* a weight average molecular weight) of less than 500 kDa. In some embodiments, a crosslinkable entity has a molecular weight *(e.g.,* a weight average molecular weight) above 500 Da. In some embodiments, a crosslinkable entity has a molecular weight *(e.g.,* a weight average molecular weight) less than 500 Da.

In some embodiments of a crosslinkable entity that comprises a polymer moiety and a crosslink moiety, the crosslink moiety contributes lipophilicity to the crosslinkable entity. In some embodiments, the identity and/or number and/or density of crosslink moieties linked to a particular polymer moiety in a crosslinkable entity is/are selected so that the crosslinkable moiety has characteristic(s) as described herein. For example, in some embodiments, a more lipophilic crosslink moiety (and/or a larger number/higher density of lipophilic crosslink moieties) will be linked to a polymer moiety when the polymer moiety is particularly hydrophilic, particularly long, and/or is characterized by particularly poor skin penetration in its unlinked state. In some embodiments, the crosslinkable entity can contain an additional moiety that does not participate in crosslinking, but contributes to the lipophilicity of the crosslinkable entity, providing improved penetration characteristics.

Without wishing to be bound by any particular theory, we propose that, in some embodiments, it may be desirable to select a particular combination of polymer moiety and crosslink moiety (and/or number and/or density thereof) so as to achieve three-dimensional packing of the crosslinkable entity within a certain volume, e.g., which may facilitate transfer through one or more skin structures or layers (e.g., rendering the crosslinkable entity sufficiently "slippery" to pass through). In some embodiments, intra- and/or inter-molecular interactions may contribute to three-dimensional packing of a crosslinkable entity. In some embodiments, three-dimensional packing of a particular cross-linkable entity as described herein occurs by self-assembly (e.g., without requiring addition of another agent), at least under certain environmental conditions (e.g., physiological conditions).

Typically, a crosslinkable entity is biocompatible. For example, in some embodiments, administration of a crosslinkable entity as described herein does not cause significant irritation and/or inflammation (*e.g*., at the site of administration and/or at the target site).

In some embodiments, a crosslinkable entity is utilized in accordance with the present invention in a form or preparation having a viscosity suitable for topical application - *e.g*., with sufficient viscosity to maintain the applied crosslinkable entity in contact with the administration site for long enough to permit penetration and/or to otherwise permit distribution to the target site rather than simply dribbling off; in some such embodiments, the viscosity is within a range that permits the applied crosslinkable entity to flow into microstructures on the skin surface. In some embodiments, a crosslinkable entity is utilized in accordance with the present invention in a form or preparation having a viscosity suitable for administration by injection. In some embodiments, a suitable viscosity is a viscosity wherein the crosslinkable entity can be applied topically without rapidly running off, and/or can be rubbed into skin.

In some embodiments, the present disclosure provides a system comprising two or more crosslinkable entities, that interact with one another to form a crosslinked material (*e.g*., to provide a crosslinked material *in situ* at a target site on, at, in or below the epidermis, dermis or underlying hypodermis).

In some embodiments, at least one crosslinkable entity of a provided system comprises a polymer moiety linked with a crosslink moiety. In some such embodiments, at least one crosslinkable entity of the system does not comprise a polymer moiety linked with a crosslink moiety (*i.e.,* comprises or consists of a crosslink moiety but not a polymer moiety); in some such embodiments, only one crosslinkable entity of a provided system comprises a polymer moiety linked with a crosslink moiety. Alternatively, in some embodiments, each crosslinkable entity of a provided system comprises a polymer moiety linked with a crosslink moiety; in some such embodiments, each crosslinkable entity of a provided system comprises the same polymer moiety.

Different crosslinkable entities of a provided system comprise complementary crosslink moieties so that the crosslinkable entities react to form a crosslinked material; in some embodiments, such crosslinking occurs absent any added catalyst.

**In** some embodiments, at least one crosslinkable entity of a provided system has a molecular weight *(e.g.,* a weight average molecular weight) above 500 daltons as described herein *(e.g.,* has a molecular weight *(e.g.,* a weight average molecular weight) within a range of 1-1000 kDa). In some embodiments, at least one crosslinkable entity of a provided system has a molecular weight *(e.g.,* a weight average molecular weight) within a range of 1-10,000 kDa as described herein. In some embodiments, at least one crosslinkable entity of a provided system has a molecular weight *(e.g.,* a weight averaged molecular weight) greater than 10,000 kDa. In some such embodiments, at least one crosslinkable entity of the system does not have a molecular weight *(e.g.,* a weight average molecular weight) above 500 daltons as described herein (*e.g*., within a range of 1-400 daltons); in some such embodiments, only one crosslinkable entity of a provided system has such a high molecular weight *(e.g.,* a weight average molecular weight). Alternatively, in some embodiments, each crosslinkable entity of a provided system has such a high molecular weight *(e.g.,* a weight average molecular weight).

### Polymer Moieties

In some embodiments, the present invention encompasses a crosslinkable entity, which comprises a polymer moiety and a crosslink moiety. In some embodiments, one or more of the polymer moieties are glycosaminoglycans or polysaccharides. In some embodiments, "polysaccharides" include dextran, starch or pectin. In some embodiments, one or more of the polymer moieties is dextran. In some embodiments, one or more of the polymer moieties is starch. In some embodiments, one or more of the polymer moieties is pectin. In some embodiments, "glycosaminoglycans" includes hyaluronic acid (HA), heparin sulfate, chondroitin sulfate, dermatan sulfate and keratin sulfate. In some embodiments, a polymer is hyaluronic acid.

In some embodiments, one or more of the polymer moieties is a synthetic polymer. In some embodiments, a "synthetic polymer" includes PEG, PEG-diamine, polyacrylic acid, N-(2-Hydroxypropyl) methacrylamide (HPMA), polycaprolactone (PCL) or poly(lactic-co-glycolic acid) (PLGA). In some embodiments, a synthetic polymer is PEG. In some embodiments, a synthetic polymer is PEG-diamine. In some embodiments, a synthetic polymer is polyacrylic acid. In some embodiments, a synthetic polymer is HPMA. In some embodiments, the synthetic polymer is PCL. In some embodiments, the synthetic polymer is PLGA.

In some embodiments, one or more of the polymer moieties is a polypeptide (e.g., a protein). In some embodiments, a useful polypeptide is or comprises collagen, gelatin, elastin, or a functional fragment thereof. In some embodiments, a polypeptide is or comprises collagen or a functional fragment thereof. In some embodiments, a polypeptide is gelatin. In some embodiments, a polypeptide is elastin.

In some embodiments, one or more polymer moieties included in a system as described herein is or comprises HA. In some embodiments, one or more polymer moieties included in a system as described herein is or comprises a glycosaminoglycan. In some embodiments, one or more polymer moieties included in a system as described herein is or comprises a polypeptide *(e.g.,* a non-natural polypeptide and/or a synthetic or recombinant polypeptide). In some embodiments, a provided system includes a plurality of different crosslinkable entities comprising polymer moieties; in some such embodiments, the polymer moieties are selected from the group consisting of HA, proteoglycans, polypeptides, and combinations thereof.

### Crosslink Moieties

As described herein, the present disclosure provides systems that include two or more crosslinkable entities that are compatible with one another in that they react to form a crosslinked material *in situ.*

Those skilled in the art are aware of a variety of chemical crosslinking systems. In many embodiments, the present disclosure utilizes crosslink chemistries that do not require an added catalyst.

In some embodiments, one or more of the crosslinkable entities of the system comprise a crosslink moiety. In some embodiments, a "crosslink moiety" is capable of participating in a click reaction.

A person of ordinary skill in the art will appreciate that a click reaction may be a reaction of two or more moieties which brings two or more substrates together and occurs under physiological pH. In some embodiments, a click reaction exhibits suitable kinetics (e.g. second-order rate constant (k2) is about 9 M-1 s -1).

For example, in some embodiments, crosslink chemistries can include, but is not limited to, cycloadditions, nucleophilic substitution reactions, condensation reactions and nucleophilic addition reactions. In some embodiments, a click reaction is a [3+2] cycloaddition, a [4+2] cycloaddition or a [4+1] cycloaddition. In some embodiments, a click reaction is an azide-alkyne cycloaddition, a nitrone-olefin cycloaddition or a Diels-Alder reaction. In some embodiments, a click reaction is a Schiff reaction, a Michael-type addition, a nucleophilic substitution reaction on a haloacetate, a formation of a disulphide linkage, a free radical polymerization, a Huysgen reaction, phenols (tyramines) that spontaneously cross link after their enzymatic oxidation to catechols or a reaction between cyanobenzothiazole (CBT) and D-cysteine (CYS). In some embodiments, a click reaction is a reaction between CBT and D-cysteine. In some embodiments, a click reaction is a reaction between CBT and L-cysteine. In some embodiments, a click reaction is a reaction between CBT and a mixture of D and L-cysteine. In some embodiments, CBT can include analogs of CBT, e.g., isotopically labelled CBT. In some embodiments, CBT includes substituted analogs of CBT. In some embodiments, CYS can include analogs of CYS, e.g., isotopically labelled CYS. In some embodiments, CYS includes substituted analogs of CYS.

In some embodiments, the present disclosure appreciates that certain crosslink moieties may be particularly useful *in situ* crosslinking contexts as described herein.

For example, in some embodiments, the present disclosure utilizes one or more crosslink moieties characterized by a desired degree of lipophilicity, for example, when linked with a particular polymer moiety. To give but one example, CBT represents a crosslinkable moiety that can be linked with a polymer moiety in a useful crosslinkable entity as described herein. Those skilled in the art, reading the present disclosure, will appreciate that, in some embodiments, lipophilicity of a crosslinkable entity comprising a particular polymer moiety may be adjusted through linkage of a plurality of hydrophobic moieties (e.g., hydrophobic crosslink moieties), which may be the same or different (and need not all be crosslink moieties).

Additionally, in some embodiments, a crosslink moiety is or comprises azide, alkyne, nitrone, olefin, diene, tetrazine, isocyanate, Michael acceptor, enone, aldehyde, amine, α-halo carbonyl moiety, maleimide, thiol, CBT, D-cysteine, acrylic residues, phenol, tyramine or catechol. In some embodiments, a crosslink moiety is or comprises D-cysteine. In some embodiments, a crosslink moiety is or comprises L-cysteine. In some embodiments, a reactive moiety is or comprises CBT.

In some embodiments, at least one crosslink moiety included within a provided system may be utilized without linkage to a polymer moiety. That is, in some embodiments, at least one crosslinkable entity included within a provided system may consist of a crosslink moiety, or may comprise a crosslink moiety and at least one other moiety that is not a polymer moiety.

In some embodiments, a provided system may utilize a crosslink moiety that is or comprises, for example, a diamine, peptide, dithiol or dihydrazide. In some such embodiments, a diamine may be an ethylene diamine, *e.g.,* a polyethylene glycol (PEG) diamine, lysine, *etc.;* in some such embodiments, a dihydrazide may be an oxalic dihydrazide, malonic dihydrazide, succinic dihydrazide, glutaric dihydrazide, adipic dihydrazide or pimelic dihydrazide, *etc.* Without wishing to indulge in semantics, it is noted that certain such compounds could be described either as consisting of a crosslink moiety, or as comprising one or more crosslink moieties (e.g., each of which may be a single reactive atom or a small number of atoms), and a polymer moiety that may include only a very small *(e.g.,* 2 or 3) number of "monomers" (and, in some instances where each such "monomer" might consist of only a few atoms). Regardless, those of ordinary skill in the art, reading the present disclosure, will appreciate that these compounds may be utilized in various embodiments, together with other crosslinking entities with complementary crosslink moieties as described herein.

### Other Moieties

In some embodiments, a crosslinkable entity as described herein may comprise one or more moieties other than a polymer moiety or crosslink moiety, which other moiety(ies) may, in some embodiments, be covalently associated with a polymer moiety and/or with a crosslink moiety. In some embodiments, an "other" moiety may be releasably associated with the crosslinkable entity (e.g., via a cleavable bond; in some such embodiments, such a cleavable bond may be cleaved at the target site).

For example, in some embodiments, a crosslinkable entity may comprise a drug moiety (which may, in some embodiments, itself be in a pro-drug form).

In some embodiments, a drug moiety is or comprises a synthetic or natural small molecule or biomolecule (e.g., a carbohydrate, a lipid, a nucleic acid, a polypeptide, or an analog or combination thereof).

In some embodiments, a drug moiety may be one that improves the appearance of the skin in one or more ways. For example, in some such embodiments, a drug moiety may brighten skin; clear breakouts; firm; improve cellular activity within skin; improve collagen synthesis; improve healing profile of a wound; improve pigmentation; improve skin barrier function; normalize healthy micoflora via topical pre-biotics; prevent damage *(e.g.,* due UV exposure); reduce the appearance of scars; reduce inflammation; reduce itching; reduce redness; seal wounds; smooth, or treat burns or combinations thereof.

In some embodiments, a drug moiety may comprise an alpha-hydroxy acid *(e.g.,* lactic, tartaric, or citric acid), an antioxidant *(e.g.,* glutathione, an isoflavone, a polyphenol *(e.g.,* resveratrol), or selenium), a beta-hydroxy acid *(e.g.,* salicylic acid), a polyhydroxyl acid *(e.g.,* gluconolactone or lactobionic acid), hydroquinone, natural skin lightening agents (e.g., Kojic acid), retinoids (e.g. retinoic acid, retinol, tretinoin or derivatives thereof), a ceramide, a peptide, an amino acid, a curcuminoid, vitamins (and derivatives thereof) (e.g., L-Ascorbic acid, vitamin B, niacinimide, and vitamin K), a sunscreen agent (*e.g*., oxybenzone, avobenzone, octisalate, octocrylene, homosalate, octinoxate, Meroxyl (SX and XL) or metallic oxides), coloring agents, pigments, or natural botanicals or combinations or prodrugs thereof.

In some embodiments, a drug moiety may comprise an anti-inflammatory (*e.g*., corticosteroids, non-steroidal anti-inflammatory drugs (crisaborole), an antibiotic (*e.g*., clindamycin or ketoconazole), an antifungal (*e.g*., clotrimazole or ketoconazole), an anti-acne agent (*e.g*. retinoids or salicylic acid), an analgesics, an anticancer or antiproliferative agent, an agent which treats erythema (*e.g*., oxymetazoline hydrochloride), an agent which reduces subdermal fat *(e.g.,* deoxycholate), a hair growth agent *(e.g.,* finasteride), or combinations or prodrugs thereof.

### Microneedling

Microneedling describes a collection of technologies that have been developed and that utilize micron sized "needles" to improve the appearance or health of skin and/or deliver active agents into skin. Traditionally used as a collagen induction therapy for facial scars and skin rejuvenation, it is also widely used as a transdermal delivery system for agents *(e.g.,* therapeutic drugs and vaccines). Microneedling has been shown to enhance the skin permeability of agents either *in vitro, ex vivo* or *in vivo.*

Microneedling provides advantages over conventional injection with hypodermic needles or cannulas. Among other things, it can reduce anxiety that patients have when confronted with conventional needles or cannulas. Unlike injections, microneedling enables faster treatment of large areas of skin. Faster treatment time can increase the revenue rate from providers and also increases convenience to patients receiving the treatment. Furthermore, microneedling may offer more uniform treatment than individual injections. Depending on the application, microneedling can reduce the demands for specific skill and technique as compared to conventional injection. Certain types of "consumer grade" microneedling devices are offered for sale directly to consumers without any particular skill in microneedling. The depth to which microneedles penetrates into skin is controlled, in part, by the length of microneedles. It has been shown that the use of short microneedles is less painful than conventional injections. Microneedling offers the ability to deliver agents into both the epidermis and dermis in a more uniform manner compared to conventional injection. This is particularly true for applications in which agents are intended to be delivered into more superficial layers of the skin (e.g., epidermis and upper dermis). By contrast, intradermal injection using hypodermal injections requires careful placement of the needle within the skin. Certain agents must be delivered within a restricted depth to produce maximal benefit. Microneedling also produces less hazardous waste and is easier to use than needles. Furthermore, microneedling can provide those who are limited in their ability to seek hospital care with the ability to safely administer drugs in the comfort of their homes. Microneedling shows lower rates of microbial invasion into delivery sites because it damages the skin to a depth of 10-15µm making it difficult for bacteria to enter the bloodstream, and giving the body a smaller wound to repair. McConville A, Hegarty C, Davis J (June 2018). "Mini-Review: Assessing the Potential Impact of Microneedle Technologies on Home Healthcare Applications". Medicines. 5 (2): 50.

In some embodiments, microneedles in accordance with the present disclosure are solid or hollow. In some embodiments, solid microneedles may be employed for creating holes in skin (e.g., stratum corneum), increasing the skin penetration of topically applied formulations. In some embodiments, solid micro needles are coated with agents (e.g., crosslinkable entities). After removal of the microneedles, agents remain deposited within the skin. In some embodiments, solid microneedles may be rolled or pushed over formulations on skin *(e.g.,* depositing formulation into skin). In some embodiments, a hollow microneedle comprises a hollow bore in the center of the needle. In some embodiments, hollow microneedles are employed to inject agents directly into a skin. In some embodiments, a hollow bore transports agents through an interior of needles *(e.g.,* by diffusion or by pressure driven flow).

In some embodiments, a microneedle device in accordance with the present disclosure includes one or more microneedles. In some embodiments, a microneedle device is an individual microneedle, a patch or a roller.

In some embodiments, a microneedle device in accordance with the present invention includes flat or round microneedles.

In some embodiments, microneedles in accordance with the present disclosure comprise a material selected from the group consisting of silicon, metal (*e.g*., stainless steel, titanium, palladium, nickel, platinum, alloys, gold), glass, ceramics (*e.g*., alumina, calcium phosphate, calcium sulphate), polymers (*e.g*., hydroxypropyl methylcellulose, hyaluronic acid, carboxymethycellulose (CMC), alginates, poly (*e.g.*, methylvinylether/maleic anhydride), polystyrene, polyvinyl alcohol, polyvinylpyrrolidone (PVP), polylactic acid, polyglycolic acid, and their co-polymers (poly (lactic-co-glycolic acid) [PLGA])), sugar (*e.g*., maltose, trehalose, raffinose, mannitol, xylitol, galactose), and combinations thereof. Without wishing to be bound by any particular theory, general properties essential to qualify as a material for microneedles include inert nature, absence of immunogenicity, high tensile strength, non-brittle nature, good mechanical strength, low corrosion rate, biocompatibility, stability, ease of availability, and low cost.

In some embodiments, microneedles comprise a biodegradable material (*e.g*., polymer, sugar, *etc.).* In such embodiments, microneedles may be dissolved and/or degraded at an administration site *(e.g.,* the removal is not required). Such microneedles may be attached to an applicator before the application, but detached from the applicator after the application.

In some embodiments, microneedles in accordance with the present disclosure have a length within a range of about 10 µm and about 1000 µm, about 10 µm and about 750 µm, about 10 µm and about 500 µm, about 100 µm and about 1000 µm, about 100 µm and about 750 µm, about 100 µm and about 500 µm, about 100 µm and about 250 µm, about 250 µm and about 750 µm, or about 250 µm and about 500 µm.

In some embodiments, microneedles in accordance with the present disclosure have a microneedle density within a range of about 10 to 15000 needles/cm², about 10 to 10000 needles/cm², about 10 to 5000 needles/cm², about 10 to 1000 needles/cm², about 50 to 15000 needles/cm², about 50 to 10000 needles/cm², about 50 to 5000 needles/cm², or about 20 to 1000 needles/cm².

In some embodiments, microneedles in accordance with the present disclosure have a base width within a range of about 10 µm to about 500 µm, about 10 µm to about 400 µm, about 10 µm to about 300 µm, about 10 µm to about 200 µm, about 25 µm to about 500 µm, about 25 µm to about 400 µm, about 25 µm to about 300 µm, about 25 µm to about 200 µm, about 50 µm to about 500 µm, about 50 µm to about 400 µm, about 50 µm to about 300 µm, or about 50 µm to about 200 µm.

In some embodiments, microneedles in accordance with the present disclosure have a tip diameter within a range of about 0.1 µm to about 100 µm, about 0.1 µm to about 50 µm, about 0.1 µm to about 25 µm, about 1 µm to about 100 µm, about 1 µm to about 50 µm, or about 1 µm to about 25 µm.

In some embodiments, hollow microneedles in accordance with the present disclosure have a lumen diameter within a range of about 10 µm to about 100 µm, or about 10 µm to about 50 µm.

Without wishing to be bound by any particular theory, the designing of microneedles may be to minimize the pain. Specific microneedles of about a couple hundred microns length were reported to be painless. Int J Pharm Tech 2010; 2(3): 329-344. It was reported that 13-times increment in needle length (i.e., 500-1500 microns) increases the pain by 7 times (i.e., 5-35% caused by hypodermic needle). If the length remains constant, an increase in number of microneedles (i.e., 620 micron long) 10 fold from 5- 50 also increases the pain by 3 folds.

### Administration

The present disclosure provides technologies through which an intradermal crosslinked material is established.

Those skilled in the art are aware of a variety of contexts in which it is desirable to achieve such establishment of an intradermal crosslinked material. For example, an intradermal crosslinked material may be used for facial rejuvenation, including three-dimensional restoration of facial volume, rebalancing facial proportions and symmetry, and reducing fine lines and wrinkles. Crosslinking can enhance the stability and durability of clinical implants.

Those skilled in the art will also be aware of various challenges associated with establishing an intradermal crosslinked material, particularly where the material may have a large and/or complex structure. For example, large, complex structures cannot penetrate skin on their own. Efforts to achieve installation by intradermal injection can present significant challenges, including difficulties achieving precise localization *(i.e.,* specifically between the epidermis and hypodermis). Reports have described very specific requirements for angle of injection *(e.g.,* specifically 5 to 15 degrees) and/or for utilized needles, *etc.* Doyle, G. R., & McCutcheon, J. A. (2015). Clinical Procedures for Safer Patient Care. Victoria, BC: BCcampus., Chapter 7.3. Furthermore, injection can be unpleasant for the subject, and can trigger wound healing responses that may have uncomfortable and/or unappealing consequences (*e.g*., scarring).

Among other things, the present disclosure builds upon insights provided in International Patent Application Publication Nos. WO2016/201382, WO2020/093022 and U.S. Patent Application Publication No. US 2018/0186900, which describe certain technologies for topical application of crosslinkable entities to achieve establishment of an intradermal crosslinked material. The present disclosure provides technologies in which topical application is combined with microneedling. The present disclosure also provides insights regarding particularly effective topical application technologies (e.g., relating to the pH, concentration [e.g., relative concentration], and/or timing with which crosslinkable entities are contacted with one another and/or with skin), including as are particularly useful in combination with microneedling, as well as insights regarding particularly effective such combination (*e.g*., relating to relative timing of application of crosslinkable entities as compared with microneedling and/or one or more parameters [e.g., needle length]) of such microneedling.

In some embodiments, at least one crosslinkable entity is administered in combination with microneedling. In some embodiments, at least one crosslinkable entity *(e.g.,* a first crosslinkable entity comprising a polymer moiety) is applied topically before microneedling. In some embodiments, at least one crosslinkable entity is applied topically after microneedling. In some embodiments, at least one crosslinkable entity is applied topically before and after microneedling. The present disclosure provides an insight that application prior to microneedling may be helpful for delivery of at least one crosslinkable entity at a deeper target site *(e.g.,* under stratum corneum, dermis). Without wishing to be bound by any particular theory, microneedling may provide physical force (*e.g*., downward pressure) to a crosslinkable entity if it is present during microneedling.

In some embodiments, a first crosslinkable entity (*e.g*., comprising polymer moiety) is applied topically (*e.g*., before and/or after microneedling). In some embodiments, a second crosslinkable entity is applied via microneedle(s) (*e.g*., second crosslinkable entity is coated on microneedles, or contained in hollow bores of microneedles).

In some embodiments, a first and second crosslinkable entities are administered simultaneously. For example, in some embodiments, first and second crosslinkable entities may be combined prior to or as they are administered. In some embodiments, such combination may be referred to as a pre-mix, and may be prepared a period of time prior to administration; typically, such a pre-mix is prepared within about 30 minute of administration.

In some embodiments, first and second crosslinkable entities are administered sequentially. In some embodiments, first crosslinkable entities are administered first *(e.g.,* in combination with microneedling). In some embodiments, second crosslinkable entities are administered first *(e.g.,* in combination with microneedling).

In some such embodiments, a period of time separates administration of the first and second crosslinkable entities. In some embodiments, a period of time between administration of first and second crosslinkable entities is sufficient to permit substantial penetration of the first-administered crosslinkable entity before the second crosslinkable entity is administered. In some embodiments, such a period of time is about 30 seconds, about 1 minute, about 2 minutes, about 3 minutes, about 4 minutes, about 5 minutes, about 10 minutes, about 30 minutes, about 1 hour, about 6 hours, about 12 hours, or more. In some embodiments, such a period of time is about 2 hours. In some embodiments, such a period of time is less than about 12 hours, less than about 6 hours, less than about 1 hour, less than about 20 minutes, less than about 10 minutes, less than about 5 minutes, less than about 2 minutes, or less than about 1 minute. In some embodiments, the period of time is sufficient so that at least about 1%, at least about 2%, at least about 5%, at least about 10%, or at least about 20% of the first-administered crosslinkable entity has penetrated prior to administration of the second-administered crosslinkable entity. In some embodiments, the period of time is sufficient such that the first agent has not degraded and/or been cleared from skin. Kinetics and/or extent of penetration for a particular crosslinkable entity may be determined, for example, as described herein, including through use of a model system (*e.g*., porcine skin); the relevant period of time may be selected in light of such determination.

The present disclosure provides an insight that certain pH ranges (*e.g*., lower than physiological pH) may allow for the increased delivery of crosslinkable entities and/or formation of an *in situ* crosslinked material. For example, certain crosslinkable entities *(e.g.,* first crosslinkable entities *(e.g.,* HA-CBT), second crosslinkable entities *(e.g.,* CEC, CKC)) react quickly at physiological pH *(e.g.,* as shown in Example 2). In some embodiments, a fast reaction may increase the size (e.g., molecular weight, physical dimension) of crosslinkable entities, so that crosslinkable entities may not have sufficient time to penetrate into a target site (e.g., below stratum corneum, epidermis, dermis). In some embodiments, a pH lower than a physiological pH may slow and/or delay a crosslinking reaction. In some embodiments, first and second crosslinkable entities may be administered at a pH within a range of 2-7, 3-6, 3-5, 4-6, or 4-5. In some embodiments, an additional additive may further slow and/or delay a crosslinking reaction.

In some embodiments, a preparation including crosslinkable entities comprises a buffer. In some embodiments, a buffer comprise citrate, acetate, MES, alphy hydroxyl acids (glycolic, lactic, tartaric), or any other buffer that can maintain a desired pH. In some embodiments, a preparation including crosslinkable entities comprises citrate at a concentration within a range of 10 to 100 mM.

In some embodiments, provided technologies achieve delivery of a sufficient amount/concentration of crosslinkable entities at a target site, so that crosslinkable entities can form a larger molecule and/or structure than initial crosslinkable entities. A smaller molecule is typically easier to be transported than a larger molecule. However, a larger molecule is preferred because it is retained at a target site longer. For example, it may take longer for a larger molecule to be decomposed and/or diffused out of a target site than a smaller molecule. Delivery of smaller (e.g., lower molecular weight) crosslinkable entities has not been preferred because an insufficient amount/concentration of smaller crosslinkable entities may not form an *in situ* crosslinked material having a large structure that retains at the target site for a sufficient time. The present disclosure provides a surprising insight that administration in combination with microneedling is particularly beneficial for a delivery of a crosslinkable entity comprising a polymer moiety having a certain molecular weight range *(e.g.,* less than 50 kDa, 40 kDa, 30 kDa, 20 kDa, 15 kDa). Without wishing to be bound by any particular theory, a sufficient amount/concentration of crosslinkable entities comprising a polymer moiety having a certain molecular weight can be delivered to a target site, when administered in combination with microneedling. In some embodiments, a sufficient concentration of crosslinkable entities to form an *in situ* crosslinked material having a large structure that retains at the target site for a sufficient time *(e.g.,* 24 hours, 3 days, 7 days, 30 days, 60 days, or 90 days) is about 5 µg/cm² to about 300 µg/cm², 5 µg/cm² to about 100 µg/cm², 5 µg/cm² to about 90 µg/cm², 5 µg/cm² to about 80 µg/cm², 5 µg/cm² to about 70 µg/cm², 5 µg/cm² to about 60 µg/cm², 5 µg/cm² to about 50 µg/cm², 5 µg/cm² to about 40 µg/cm², 5 µg/cm² to about 30 µg/cm², 5 µg/cm² to about 20 µg/cm², 5 µg/cm² to about 10 µg/cm², 10 µg/cm² to about 100 µg/cm², 10 µg/cm² to about 90 µg/cm², 10 µg/cm² to about 80 µg/cm², 10 µg/cm² to about 70 µg/cm², 10 µg/cm² to about 60 µg/cm², 10 µg/cm² to about 50 µg/cm², 10 µg/cm² to about 40 µg/cm², 10 µg/cm² to about 30 µg/cm², 10 µg/cm² to about 20 µg/cm², 20 µg/cm² to about 100 µg/cm², 20 µg/cm² to about 90 µg/cm², 20 µg/cm² to about 80 µg/cm², 20 µg/cm² to about 70 µg/cm², 20 µg/cm² to about 60 µg/cm², 20 µg/cm² to about 50 µg/cm², 20 µg/cm² to about 40 µg/cm², or 20 µg/cm² to about 30 µg/cm².

The present disclosure provides an insight that certain concentration ranges of crosslinkable entities may allow for the increased delivery of crosslinkable entities and/or formation of an *in situ* crosslinked material. For example, in some embodiments, a high concentration of crosslinkable entities may increase viscosity and/or reduce penetration of crosslinkable entities. In some embodiments, a high concentration of crosslinkable entities may not penetrate into a target site *(e.g.,* under epidermis *(e.g.,* stratum corneum), dermis) via a conventional administration/delivery. In some embodiments, provided technologies (e.g., administration in combination with microneedling) allows for the penetration of a high concentrated crosslinkable entities into a target site *(e.g.,* under epidermis *(e.g.,* stratum corneum), dermis). In some embodiments, a low concentration of crosslinkable entities may not provide a sufficient amount of crosslinkable entities to form an *in situ* crosslinked material. In some embodiments, a concentration of a crosslinkable moiety is within a range of 0.01 to 100 mg/mL, 0.1 to 100 mg/mL, 1 to 100 mg/mL, or 1 to 10 mg/mL.

In some embodiments, provided crosslinkable entities are administered to a subject's face *(e.g.,* full face and/or specific targets of a subject's face such as to lips, lower lip, upper lip, tear troughs, crow's feet, nasolabial folds, forehead, cheeks or combinations thereof). In some embodiments, provided crosslinkable entities are administered to a non-facial site *(e.g.,* knees, neck, décolletage, legs, arms, torso, buttocks or feet). In some embodiments, provided crosslinkable entities are administered to hands *(e.g.,* to the back of a hand). In some embodiments, provided crosslinkable entities are administered to ear lobes.

In some embodiments, a site of administration is prepared prior to administration of crosslinkable entities. In some embodiments, a site of administration is prepared by washing the site with tepid water and soap. In some embodiments, a site of administration is prepared through tape stripping.

In some embodiments, a site of application is covered after application of crosslinkable entities. In some embodiments, a site of application is covered with Tegaderm^{™} type film after application of crosslinkable entities.

In some embodiments, skin will be treated with water after administration of a system.

In some embodiments, crosslinkable entities are administered daily. In some embodiments, crosslinkable entities are administered at least once daily. In some embodiments, crosslinkable entities are administered at least twice daily. In some embodiments, crosslinkable entities are administered a 1-5 times daily. In some embodiments, crosslinkable entities are administered a 3-5 times daily. In some embodiments, crosslinkable entities are administered every 3 days. In some embodiments, crosslinkable entities are administered every 7 days. In some embodiments, crosslinkable entities are administered about every 15 days. In some embodiments, crosslinkable entities are administered about every 30 days. In some embodiments, crosslinkable entities are administered about every 60 days. In some embodiments, crosslinkable entities are administered about every 90 days.

In some embodiments, a chemical entity which improves skin penetration for a crosslinkable entity may be administered. In some embodiments, the chemical entity is administered simultaneously with a crosslinkable entity. In some embodiments, the chemical entity and the crosslinkable entity are administered at different times.

In some embodiments, one or both of a penetration inhibitor (e.g., which may interact with and/or otherwise retard penetration of a crosslinkable entity) and/or a cross-link inhibitor (e.g., which may block one or more features of a crosslinkable entity or crosslink moiety, or otherwise interfere with reaction of crosslink moieties to generate a crosslink) may be administered. In some such embodiments, such an inhibitor may be removed (e.g., via diffusion, washing, degradation) prior to, during, upon or after administration of the crosslinkable entity.

In some embodiments, one or more crosslinkable entities is administered as or in a sustained-release formulation. In some embodiments, one or more crosslinkable entities is encapsulated within a matrix or particle *(e.g.,* a nanoparticle). In some embodiments, one or more crosslinkable entities is provided as or in an emulsion or dispersion. In some embodiments, additives are added to a formulation to retard the degradation of crosslinkable entity.

In some embodiments, crosslinkable entities whose crosslink moieties interact to form crosslinks in the crosslinked material are administered in relative amounts (e.g., are formulated for administration in relative amounts) so that complementary crosslink moieties are present in stoichiometric amounts. Alternatively, in some embodiments, such crosslinkable entities are administered in relative amounts so that one of a pair of complementary crosslink moieties is present in molar excess relative to the other; in some such embodiments, such molar excess is within a range of 1.1:1 - 10,000:1. In some embodiments, molar excess of the relative amounts of crosslinkable moieties is within a range of 1.1:1 - 2:1. In some embodiments, molar excess of the relative amounts of crosslinkable moieties is within a range of 1.1:1 - 10:1. In some embodiments, molar excess of the relative amounts of crosslinkable moieties is within a range of 1.1:1 - 100:1. In some embodiments, molar excess of the relative amounts of crosslinkable moieties is within a range of 1.1:1 - 1,000:1.

In some embodiments, a formulation comprises about 0.001% w/w to about 5.00% w/w of a crosslinkable entity. In some embodiments, a formulation comprises about 0.01% w/w to about 5.00% w/w of a crosslinkable entity. In some embodiments, a formulation comprises about 0.1% w/w to about 5.00% w/w of a crosslinkable entity. In some embodiments, a formulation comprises about 1% w/w to about 5.00% w/w of a crosslinkable entity. In some embodiments, a formulation comprises about 1 % w/w to about 3% w/w of a crosslinkable entity. In some embodiments, a formulation comprises about 2% w/w of a crosslinkable entity. In some embodiments, a formulation comprises PBS and about 2% w/w of a crosslinkable entity.

In some embodiments, crosslinkable entities may be administered via microneedles. For example, in some embodiments, crosslinkable entities are coated on microneedles. In some embodiments, crosslinkable entities are contained in a hollow bore of microneedles. In such embodiments, crosslinkable entities may be administered topically as well. In some embodiments, first crosslinkable entities are administered topically and second crosslinkable entities may be administered via microneedles. In some embodiments, second crosslinkable entities are administered topically and first crosslinkable entities may be administered via microneedles.

In some embodiments, a microneedle device is a dermaroller. In some embodiments, provided technologies include rolling a dermaroller. In some embodiments, each rolling includes 1, 2, 3, 4, 5, 6, 7, 8, 9, 10, 11, 12, 13, 14, 15, 16, 17, 18, 19, or 20 passes. In some embodiments, a dermaroller is rolled 1, 2, 3, 4, 5, 6, 7, 8, 9, or 10 times. In some embodiments, a dermaroller is rolled over the same area with multiple passes in different directions.

In some embodiments, one or more crosslinkable entities may be injected via microneedles (e.g., that may be associated with a patch). For example, in some embodiments, one or more crosslinkable entities, or combinations thereof may be administered through microneedles (e.g., as a liquid); alternatively or additionally, in some embodiments, one or more crosslinkable entities, or combinations thereof, may be disposed within one or more microneedles, which may dissolve or degrade upon or after application. Those skilled in the art will appreciate that use of dissolving or degrading microneedle(s) may be particularly useful or desirable for delivery of relatively viscous materials (e.g., partially or wholly formed crosslinked material as described herein). In some embodiments, one or more crosslinkable entities may be disposed in a microneedle; *e.g.,* in a lumen *(e.g.,* bore) thereof; in some such embodiments, the one or more crosslinkable entities (or a crosslinked material formed therefrom) may be released by injection through the lumen, and/or by dissolution or degradation of the microneedle (e.g., of its walls). In some embodiments, one or more crosslinkable entities may be integrally formed with a microneedle; in some such embodiments, the microneedle may be otherwise formed of a material that dissolves or degrades upon or after application, thereby releasing the one or more crosslinkable entities, or a crosslinked material formed therefrom.

In some embodiments, skin has been pretreated prior to *(e.g.,* promptly or immediately prior to) administration of one or more crosslinkable entities. In some embodiments, skin pretreatment is accomplished by administration or application of a permeabilizing agent or device. In some embodiments, skin pretreatment comprises one or more of application of an abrasive cleanser or chemical peel, dermablation, electroporation, iontophoresis, low-frequency sonophoresis, mirconeedling. In some embodiments, skin is abraded prior to administration of a system. In some such embodiments skin is abraded with microneedles and/or fraction lasers prior to administration of the system. In some embodiments, a crosslinkable entity will be administered after (e.g., promptly or immediately after) pretreatment of skin with microneedles.

In some embodiments, one or more crosslinkable entities may be injected by a dual bore syringe or needle. In some such embodiments, first and second crosslinkable entities are maintained in separate compartments of the syringe or needle at least until administration. In some embodiments, they are combined during administration; in other embodiments, they are maintained separately during administration (i.e., each is separately administered, optionally at times separated by a time period as described herein). In some embodiments, a dual bore syringe or needle further comprises an acid reservoir. In some embodiments, an acid reservoir may provide a pH within a range of 3-5 before, during and/or after administration of one or more crosslinkable entities.

In some embodiments, provided technologies include a preparation of crosslinkable entities. In some embodiments, a preparation is made by dissolving dry crosslinkable entities in a solvent or a solvent system. In some embodiments, a preparation is made by dissolving crosslinkable entities into water. In some embodiments, preparation of an aqueous solution also involves pH adjustment *(e.g.,* using buffers, NaOH, *etc.),* and/or application of disruptive energy and/or force such as, e.g., sonication, and/or homogenization.

### Characterization

Those skilled in the art, reading the present specification, will appreciate that it may be desirable to characterize one or more features of crosslinkable entities, and/or of components or combinations thereof and/or of crosslinked material(s) they produce, for example when designing (e.g., selecting appropriate components of) or producing a provided system and/or when monitoring or assessing a preparation thereof. Alternatively or additionally, in some embodiments, it may be desirable to assess one or more features of a provided system as administered, for example in order to monitor a subject or treatment thereof.

**In** some embodiments, first and second crosslinkable entities when combined *in vitro* under physiological conditions, the first and second crosslinkable entities react with one another to form a crosslinked material. Properties of such crosslinked material can be modulated through selection of the crosslinkable entities that generate it, and can represent characteristic traits of the particular crosslinked material provided by the present invention. In some embodiments, a storage modulus of a crosslinked material is within a range of 50 Pa - 10 kPa. In some embodiments, a storage modulus of a crosslinked material is within a range of 50 Pa - 1 kPa. In some embodiments, a storage modulus of a crosslinked material is within a range of 50 Pa - 500 Pa. In some embodiments, a storage modulus of a crosslinked material is within a range of 50 Pa - 100 Pa. In some embodiments, a storage modulus of a crosslinked material is within a range of 100 Pa - 10 kPa. In some embodiments, a storage modulus of a crosslinked material is within a range of 500 Pa - 10 kPa. In some embodiments, a storage modulus of a crosslinked material is within a range of 1 kPa - 10 kPa. In some embodiments, a storage modulus of a crosslinked material is within a range of 5 kPa - 10 kPa. In some embodiments, a storage modulus of a crosslinked material is within a range of 500 Pa - 5 kPa. In some embodiments, a storage modulus of a crosslinked material is within a range of 500 Pa - 1 kPa. In some embodiments, a molecular weight (e.g., weight averaged molecular weight) of a crosslinked material is greater than a molecular weight (e.g., weight average molecular weight) of a first crosslinkable entity. In some embodiments, a molecular weight (e.g., weight averaged molecular weight) of a crosslinked material is two, three, four, five, six, seven, eight, nine or ten times greater than a molecular weight (e.g., weight averaged molecular weight) of a first crosslinkable entity. In some embodiments, a molecular weight (e.g., weight averaged molecular weight) of a crosslinked material is greater than a molecular weight (e.g., weight averaged molecular weight) of a second crosslinkable entity. In some embodiments, a molecular weight (e.g., weight averaged molecular weight) of a crosslinked material is two, three, four, five, six, seven, eight, nine or ten times greater than a molecular weight (e.g., weight averaged molecular weight) weight of a second crosslinkable entity. In some embodiments, a molecular weight (e.g., weight averaged molecular weight) of a crosslinked material is measured by Gel Permeation Chromatography (GPC) analysis.

**In** some embodiments, mass loss of a crosslinked material by degradation of less than 20% over 3 days in physiological buffer. In some embodiments, mass loss of a crosslinked material by degradation of less than 10% over 3 days in physiological buffer. In some embodiments, mass loss of a crosslinked material by degradation of less than 5% over 3 days in physiological buffer. In some embodiments, a molecular weight of a crosslinked material is reduced slower (two, three, four, five, six, seven, eight, nine or ten times slower) when exposed to degradation conditions (i.e. hyaluronidase or oxidative stress) than first or second crosslinkable entities.

In some embodiments, a degree of crosslinked material swelling upon hydration is a characteristic property of a crosslinked material. In some embodiments, the degree of crosslinked material swelling will be measured based on the mass ratio between dried crosslinked material and fully swollen crosslinked material which has reached equilibrium with an external aqueous buffer (Q = mswollen / mdry). In some embodiments, a ratio between dried crosslinked material and fully swollen crosslinked material will be within a range of 50-1000. In some embodiments, a ratio between dried crosslinked material and fully swollen crosslinked material will be within a range of 100-1000. In some embodiments, a ratio between dried crosslinked material and fully swollen crosslinked material will be within a range of 500-1000. In some embodiments, a ratio between dried crosslinked material and fully swollen crosslinked material will be within a range of 750-1000. In some embodiments, a ratio between dried crosslinked material and fully swollen crosslinked material will be within a range of 50-100. In some embodiments, a ratio between dried crosslinked material and fully swollen crosslinked material will be within a range of 50-250. In some embodiments, a ratio between dried crosslinked material and fully swollen crosslinked material will be within a range of 50-500. In some embodiments, a ratio between dried crosslinked material and fully swollen crosslinked material will be within a range of 50-750. In some embodiments, a ratio between dried crosslinked material and fully swollen crosslinked material will be within a range of 250-750. In some embodiments, a ratio between dried crosslinked material and fully swollen crosslinked material will be within a range of 400-600.

In some embodiments, a first and second crosslinkable entities form a crosslinked material *(e.g.,* gel) when the dynamic storage modulus (G') becomes larger than the loss modulus (G"). In some embodiments, a first and second clickable entity will form a crosslinked material within 1 second. In some embodiments, a first and second clickable entity will form a crosslinked material within 10 seconds. In some embodiments, a first and second clickable entity will form a crosslinked material within 1 minute. In some embodiments, a first and second clickable entity will form a crosslinked material within 10 minutes. In some embodiments, a first and second clickable entity will form a crosslinked material within 30 minutes. In some embodiments, first and second clickable entity will form a crosslinked material within 60 minutes.

In some embodiments, first and second crosslinkable entities, when combined *in vitro* under physiological conditions, react with one another to form a crosslinked material. Properties of such crosslinked material can be modulated through selection of the crosslinkable entities that generate it, and can represent characteristic traits of the particular crosslinked material provided by the present invention. In some embodiments, a storage modulus of a crosslinked material is within a range of 50 Pa - 10 kPa.

### In vitro Characterization

In some embodiments, characterization may involve application of a provided system to a model *ex vivo* skin system *(e.g.,* to porcine skin or human skin).

In some embodiments, characterization may involve monitoring one or more features of skin penetration. In some embodiments, the amount of a crosslinkable entity which penetrates the skin 30 min-2 days after topical application on excised skin will be quantified, for example, after solubilizing the skin using enzymes and/or other solubilizing agents. In some embodiments, the amount of a crosslinkable entity which penetrates the excised skin at least 30 min after topical application on excised skin will be quantified after solubilizing the skin using enzymes and/or other solubilizing agents. In some embodiments, the amount of a crosslinkable entity which penetrates the skin less than 2 days after topical application on excised skin will be quantified after solubilizing the skin using enzymes and/or other solubilizing agents. In some embodiments, the amount of a crosslinkable entity that comprises a polymer moiety and a crosslink moiety which penetrates after topical application on excised skin exceeds the amount of an entity which comprises the same polymer moiety and does not comprise the crosslink moiety. In some embodiments, a crosslinkable entity comprises HA and the amount of crosslinkable entity which penetrates after topical application on excised skin will exceed the amount of natural HA at the same molecular weight (e.g., weight average molecular weight) which penetrates the skin. Preferably, in some embodiments, the amount of the crosslinkable entity that comprises HA which penetrates after topical application on excised skin will exceed the natural amount of HA already found in the skin (natural amount of HA already found in porcine skin = ~100-800 µg/g dry tissue).

In some embodiments, the depth of penetration of crosslinked material within the excised skin is measured by shining ultraviolet light on the target site. In some embodiments, the presence of crosslinked material in the porcine skin will be determined by observation of fluorescence upon shining ultraviolet light on the target site.

In some embodiments, the presence of a crosslinked material at a target site will be observable after a period of time *(e.g.,* 1, 2, 3, 4, 5, 6, 7, 8, 9, 10, 11, 12, 13, 14, 15, 20, 25, 30 or more days). In some embodiments, the presence of a crosslinked material at a target site will be observable after about 3.5 days. In some embodiments, the presence of a crosslinked material at a target site will be observable after about 9 days.

In some embodiments, presence of a crosslinked material at a target site is observed by cryosectioning followed by fluorescent microscopy. In some embodiments, presence of a crosslinked material at a target site is observed by histological staining.

### In vivo Characterization

Upon administration of the system described herein, attributes of the target site and/or site of administration, i.e. skin, will be evaluated at an appropriate time period for improvement as compared to the skin attribute prior to administration of the system. In some embodiments, skin attributes include wrinkles, radiance, firmness, moisture content, skin thickness, elasticity, and skin smoothness. In some embodiments, improvement includes repair to atrophic skin or scars. In some embodiments, the administration of the system results in fibroblast activation and/or enhanced collagen synthesis within the target sight of the skin.

In some embodiments, improvement of the skin will be measured by the Global Aesthetic Improvement Scale (GAIS). A person of skill in the art will appreciate the GAIS can be determined using the following rating scale:
- 1 = very much improved
- 2 = much improved
- 3 = improved
- 4 = no change
- 5 = worse

In some embodiments, a majority of subjects will observe > 1 improvement on the GAIS. In some embodiments, a majority of subjects will observe > 2 improvements on the GAIS. In some embodiments, a majority of subjects will observe > 3 improvements on the GAIS. In some embodiments, a majority of subjects will observe about 1 to about 2 improvements on the GAIS. In some embodiments, a majority of subjects will observe about 1 to about 3 improvements on the GAIS. In some embodiments, a majority of subjects will observe about 1 improvement on the GAIS. In some embodiments, a majority of subjects will observe about 2 improvements on the GAIS. In some embodiments, a majority of subjects will observe about 3 improvements on the GAIS.

In some embodiments, improvement in skin will be measured using the Modified Fitzpatrick Wrinkle Scale (MFWS). A person of skill in the art will appreciate the MFWS can be determined using the following rating scale:
- Class 0 - No wrinkle. No visible wrinkle; continuous skin line.
- Class 0.5 - Very shallow yet visible wrinkle.
- Class 1 - Fine wrinkle. Visible wrinkle and slight indentation.
- Class 1.5 - Visible wrinkle and clear indentation. 1-mm wrinkle depth.
- Class 2 - Moderate wrinkle. Clearly visible wrinkle, 1- to 2-mm wrinkle depth.
- Class 2.5 - Prominent and visible wrinkle. More than 2-mm and less than 3-mm wrinkle depth.
- Class 3 - Deep wrinkle. Deep and furrow wrinkle; more than 3-mm wrinkle depth.

In some embodiments, a subject's wrinkles will decrease at least 0.5 on the MFWS. In some embodiments, a subject's wrinkles will decrease between 1 and 0.5 on the MFWS. In some embodiments, a subject's wrinkles will decrease between 1.5 and 0.5 on the MFWS. In some embodiments, a subject's wrinkles will decrease between 2 and 0.5 on the MFWS. In some embodiments, a subject's wrinkles will decrease of between 2.5 and 0.5 on the MFWS. In some embodiments, a subject's wrinkles will decrease between 3 and 0.5 on the MFWS.

In some embodiments, a subject's skin will be assessed for improvements in smoothness, radiance or firmness.

In some embodiments, moisture content of the skin can be monitored. In some embodiments, moisture content of the skin will be higher as compared to untreated skin as determined by methods known in the art. In some embodiments, moisture content of the skin will be determined by Corneometer CM 825. In some embodiments, moisture content of skin will be >20% higher after administration of the system at day 7.

In some embodiments, a provided system is used to treat a person exhibiting abnormal transepidermal water loss (TEWL). A person of skill in the art will understand that abnormal TEWL can be the result of skin damage caused by, for example, burns, certain chemicals, pathological conditions (e.g. eczema), physical abrasion, tape stripping, ultraviolet radiation, or combinations thereof. In some embodiments, the level of TEWL of treated skin will be lower as compared to untreated skin as determined by methods known in the art. In some embodiments, the level of TEWL of treated skin will be statistically lower as compared to a baseline level as understood by methods known in the art. In some embodiments, TEWL will be measured by Tewameter TM 300 meter (Courage-Khazaka Electronics). In some embodiments, TEWL will be measured by Tewameter TM Nano (Courage-Khazaka Electronics). In some embodiments, TEWL will be measured by Tewameter Triple TM 330T (Courage-Khazaka Electronics). In some embodiments, TEWL will be measured by Invitro Tewameter VT310 (Courage-Khazaka Electronics). In some embodiments, TEWL of treated skin will be >10% lower after administration of the system at day 7.

In some embodiments, the skin will assessed for improvements in skin smoothness. In some embodiments, the skin after administration of the system will be smoother than skin prior to administration of the system. In some embodiments, the skin's smoothness will be assessed using Surface Evaluation of Living Skin (SELS). In some embodiments, the skin will be assessed using phaseshift rapid *in vivo* measurement of skin (PRIMOS). In some embodiments, the skin will be assessed by 3D Skin profilometry via Canfield Primos. In some embodiments, the skin will be assessed using the Lemperle wrinkle scale. In some embodiments, the skin will be assessed using a 7-point subject satisfaction scale. In some embodiments, the skin will be assessed using the Oral commissures. In some embodiments, the skin will be assessed using Allergan Skin Roughness scale.

In some embodiments, the skin will be assessed to determine the presence and/or extent of crosslinked material within the skin. In some embodiments, the presence of crosslinking material within the skin will be measured using near infrared (NIR) spectroscopy, confocal microscopy, a integrating sphere spectrophotometer, or will be determined using Viscoelastic deformation (VED, mm), elastic deformation (ED, mm), ultimate deformation (UD, mm), and pressure-deformation ratio methods. In some embodiments, the presence and/or of crosslinked material within the skin is measured by shining ultraviolet light on the target site. In some embodiments, the presence of crosslinked material in the skin will be determined by observation of fluorescence upon shining ultraviolet light on the target site.

In some embodiments, the presence of the crosslinked material at the target site will be observable after a period of time *(e.g.,* 1, 2, 3, 4, 5, 6, 7, 8, 9, 10, 11, 12, 13, 14, 15, 20, 25, 30 or more days). In some embodiments, the presence of the crosslinked material at the target site will be observable after about 2 days. In some embodiments, the presence of the crosslinked material at the target site will be observable after about 9 days. In some embodiments, the presence of the crosslinked material at the target site will be observable after about 11 days. In some embodiments, the presence of the crosslinked material at the target site will be observable after about 20 days. In some embodiments, the presence of the crosslinked material at the target site will be observable after about 30 days.

### Kits

In some embodiments, the present disclosure provides kits that comprise systems described herein.

In some embodiments, a provided kit comprises a plurality of containers or vessels, including containers that separately house first and second crosslinkable entities.

In some embodiments, a provided kit comprises at least one container or vessel that includes a plurality of separate compartments (e.g., a dual-bore syringe or needle - or dual chamber package with a mixing chamber prior to dispensing); in some embodiments first and second crosslinkable entities are separately housed in such compartments.

In some embodiments, the present disclosure provides one or more containers, vessels, or compartments in which a first or second crosslinkable entity as described herein is disposed. In some such embodiments, the disposed crosslinkable entity is present in dry form; in some such embodiments, the crosslinkable entity is present in liquid form. In some embodiments, the disposed crosslinkable entity has been stored for a period of time *(e.g.,* for at least 1 day, 1 week, 1 month, 3 months, 6 months or more); in some such embodiments, the stored composition has been stable over the period of storage time, in that at least about 80%, 85%, 90%, 95%, 96%, 97%, 98%, 99% or more of the crosslinkable entity remains undegraded. In some embodiments, the stored composition has been stable over the period of storage time, in that at least about 50%, 65%, 70%, 75% or more of the crosslinkable entity remains undegraded.

### Exemplification

The present Examples describe, among other things, certain strategies that may be used to characterize and/or assess penetrating agents (and/or components and/or composition or combinations thereof) as described herein. Such strategies (or their equivalents as will be appreciated by those skilled in the art reading the present disclosure) may be used to assess penetrating agents, components (*e.g*., moieties), compositions, or combinations thereof for suitability for use in accordance with the present disclosure. In some embodiments, therefore, the present disclosure provides technologies for characterizing and/or selecting useful moieties, linkers, penetrating agents, and/or components, compositions, and/or combinations thereof.

### Example 1: Enhanced Delivery of HA-CBT with microneedling (preparatory example, not according to the invention as claimed)

The present Example documents improvements to transdermal delivery of certain crosslinkable entities *(i.e.,* CBT-conjugated HA of two different molecular weights) when skin is microneedled, and particularly when microneedling is performed after topical application of the crosslinkable entity. Among other things, the present Example documents improvements achieved even relative to tape-stripping.

Hyaluronic acid (HA) was functionalized with a 6-amino-2-cyanobenzothiazole (CBT) derivative (e.g., conjugated to glycine). HA-gly-CBT was further derivatized with an IR dye (CF 647 amine, Sigma Aldrich) for quantitative studies. Both 10 kDa (HA₁₀CBT and HA₁₀CBT_{IR}) and 20 kDa (HA₂₀CBT_{IR}) HA derivatives were synthesized by starting the HA modification with different molecular weight HA.

Dermatomed human skin tissues, obtained from the abdomen of female donors aged approximately 40-50 years were purchased from ZenBio (North Carolina). The skin surface was cleaned with PBS and soap solution, followed by a thorough rinse with PBS once again. The tissue was then cut to pieces with a surface area of at least 2 cm² and hydrated in warm PBS for at least 30 minutes. The hydrated pieces were then allowed to dry at ambient air conditions for about 10 min. Each skin section was stretched and fixed with needles on a rigid base prior to microneedling with a dermaroller having a microneedle length of 250 µm. The concentration, volumes and the formulation used for the polymer in each example are presented in Table 1. The formulation to be tested was applied on the skin surface before or after treating the skin with the dermaroller.

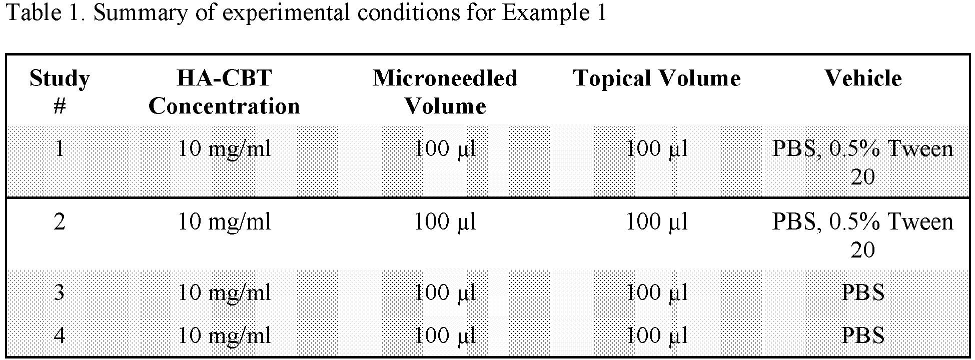

Any excess solution left on the surface, was then rubbed into the skin using a spatula. For examples where the skin surface was tape-stripped prior to topical application, an adhesive tape was applied with pressure on the skin surface and removed 20 successive times to remove much of the stratum corneum. The skin sections were then mounted on a Franz diffusion cell with a diffusion area of 1 cm² (PermeGear, Bethlehem, PA). The donor chamber was loaded with 100 µl of the formulation and sealed with parafilm. The receiver compartment was filled with 6 mL of PBS buffer (pH 7.4). Care was taken to eliminate bubbles on the skin underside and in the receptor solution. Depending on the study, the cells were maintained at a temperature of 37 °C for 1 hour or overnight.

Following incubation, each skin piece was removed from the Franz diffusion cell and rinsed with PBS. The sections were then subjected to the tape-stripping method. An adhesive tape ((Scotch^{®} Transparent Tape, 3M Corporate, St. Paul, MN) was used to strip the stratum corneum. The first 10 strips are referred to the upper stratum corneum (USC) and the next 10 strips referred to as the lower stratum corneum (LSC). A sterile surgical blade was then used to separate the epidermis from the dermis. The remaining dermis was cut into smaller pieces. The USC and the LSC were collected and placed in separate glass vials with 3 ml of PBS/methanol (1:1) mixture. The epidermis and the dermis were collected and placed in separate glass vials with 1 ml of PBS:methanol (1:1) mixture. The vials with the separated layers were then left to shake overnight at 37 °C to extract the 'IR' labeled polymers (HA-CBT_{IR}). The tissue extracts were then centrifuged to remove the tissue debris and supernatants were collected for analysis with a fluorescence plate reader (Tecan) at an excitation emission wavelength of (630 nm / 665 nm).

### Study 1. Topical delivery of HA-CBT with surface dermabrasion vs microneedling

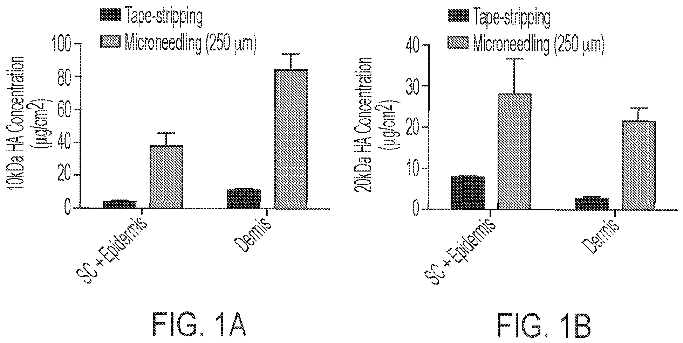

200 µl of HA₁₀CBT_{IR} or HA₂₀CBT_{IR} was applied topically to the tape-stripped skin. For the microneedled groups, 50 µl of HA₁₀CBT_{IR} or HA₂₀CBT_{IR} was applied each time before rolling with 5 passes twice. This was followed by topical application of 100 µl of the formulation on the microneedled skin. All groups were left for overnight incubation at 37 °C. Compared to the tape-stripped skin, microneedling enhanced the delivery of HA₁₀CBT_{IR} by about 9.5-fold within the stratum corneum and the epidermis and by about 8-fold within the dermis as shown in FIG. 1A. For HA₂₀CBT_{IR}, microneedling enhanced its delivery by ~4-fold within the stratum corneum and the epidermis and by ~7-fold within the dermis as shown in FIG. 1B.

### Study 2. Topical delivery of different size HA-CBT with microneedling

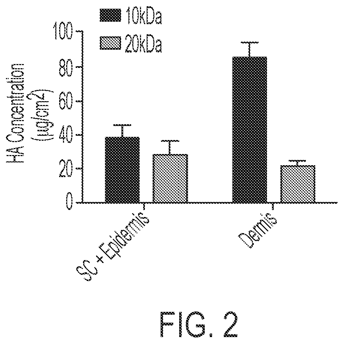

The impact of molecular weight (10 kDa vs 20 kDa) on HA-CBT delivery with microneedling was examined. 50 µl of HA-CBT was applied each time before rolling with 5 passes twice. This step was followed by topical application of 100 µl of the formulation on the microneedled skin. All groups were left for overnight incubation at 37 °C. Compared to microneedling a high molecular weight HA-CBT (HA₂₀CBT_{IR}), dermal levels of the low molecular weight HA-CBT (HA₁₀CBT_{IR}) increased by about 4-fold as shown in FIG. 2.

### Study 3. Applying HA-CBT formulation before or after microneedling

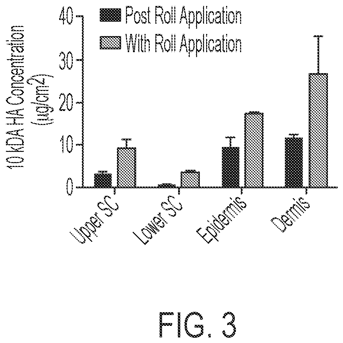

HA₁₀CBT_{IR} formulations were applied on the skin in two different ways. For the first study group, the skin surface was first microneedled with a 250 µm dermaroller at a total of ten passes. 100 µl of the formulation was then applied on the microneedled skin topically. For the second study group, 50 µl of the formulation was applied each time before rolling with 5 passes twice. 100 µl of the formulation was then applied on the microneedled skin topically. All groups were left for one-hour incubation at 37 °C. Applying HA₁₀CBT_{IR} before microneedling increased its accumulation by about 4-fold within the stratum corneum; and by about 2-fold within the epidermis and the dermis as shown in FIG. 3. These results indicate that a significant amount of material is being pushed into the skin during the microneedling process.

### Study 4. One-hour incubation vs overnight incubation

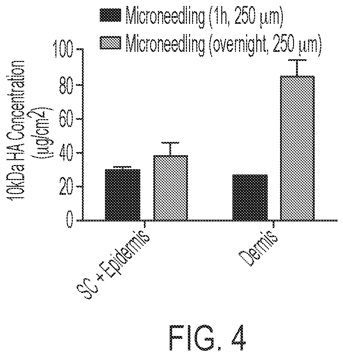

50 µl of HA₁₀CBT_{IR} formulations were applied each time before microneedling with 5 passes twice. This step was followed by applying 100 µl of the formulation on the microneedled skin topically. One group was incubated at 37 °C for an hour, and the other group was incubated overnight. Overnight incubation and 1 hour incubation resulted in similar HA-CBT accumulation within the stratum corneum and epidermis. A 3-fold increase in delivery of HA-CBT was detected in the dermis for the polymer group that was incubated overnight as shown in FIG. 4. Although less than overnight incubation, the 1 hour incubation still resulted in the delivery of significant quantities of I-IA-CBT.

### Study 5. Impact of HA-CBT concentration on delivery with microneedling

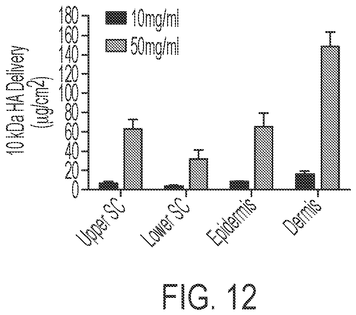

HA₁₀CBT_{IR} was applied onto skin with microneedling at concentrations of 10 mg/mL and 50 mg/mL in PBS. Details of the application are consistent with the procedure described in Example 1, Study 4 with overnight incubation. As shown in FIG. 12, the delivery of HA-CBT is 5-fold higher in the SC and epidermis with the higher concentration formulation. Similarly, the delivery of HA-CBT into the dermis is 8-fold higher. These results demonstrate HA-CBT concentration can be used as a variable to manipulate the amount of HA-CBT which is delivered when applied with microneedling.

### Example 2: Parameters for crosslinking of HA-CBT (preparatory example, not according to the invention as claimed)

The present Example demonstrates impact of crosslinkable entity concentration on production and size of produced crosslinked material *in vitro.* Among other things, the present Example defines particularly useful concentration(s) (e.g., of crosslinkable entities) and molar ratios *(e.g.,* of crosslink moieties and/or crosslinkable entities).

The present Example further documents a relationship between concentration and timing of exposure (e.g., contact) of crosslinkable entities (and/or of crosslink moieties) to one another with respect to extent and/or size of crosslinked material formed *in situ.*

Still further, the present Example documents impact of the pH at which crosslinkable entities (and/or crosslink moieties) are exposed to one another on extent and/or size of crosslinked material formed *in situ,* as well as a relationship between such pH and concentration and/or time of exposure of the relevant entities (and/or moieties).

For this example, Gel Permeation Chromatography (GPC) was used to characterize the degree of crosslinking by measuring the increase in molecular weight of the starting HA-CBT. GPC analysis was performed on an Agilent 1100 HPLC using a Diode-Array Detection (DAD) detector (326 nm), with an Agilent PL aquagel-OH MIXED-M column (8um 300 x 7.5 mm) and an Agilent PL aquagel-OH 20 (8um 300 x 7.5mm) column in series. A mobile phase of 0.2 M NaNO₃ in 9:1 water:methanol was used at a flow rate of 1 mL/min.

### Study 1. Impact of HA-CBT and crosslinker concentrations

HA₁₀CBT was mixed with the crosslinker molecule Cysteine-Lysine-Cysteine (CKC) in PBS at different concentrations and molar ratios. The two components were incubated at 37 °C for 30 min to allow for crosslinking to occur. After incubation, all samples were further diluted in PBS to a concentration of 1 mg/mL, unless the starting concentration was already below, and then run on GPC.

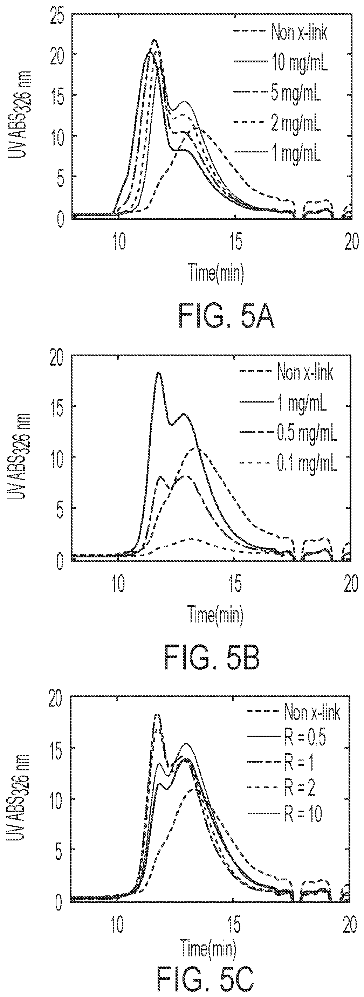

In FIGS. 5A and 5B, the concentration of HA₁₀CBT was varied from 0.1 to 10 mg/mL, with the concentration of CKC varied linearly to maintain a 1:1 CBT:Cys molar ratio. As the concentration of the polymer was increased, the chromatograph shifted to the left, indicating the formation of larger crosslinked structures. Concentrations of HA₁₀CBT above 20 mg/mL can lead to the formation of higher molecular weight, insoluble gels. As shown in FIG. 5B, the shift in retention time was much less for samples which were crosslinked below 1 mg/mL, indicating that HA₁₀CBT concentrations above 1 mg/mL are preferable for forming larger crosslinked structures.

In FIG. 5C, the concentration of HA₁₀CBT was kept constant at 1 mg/mL and mixed with different concentrations of CKC leading to CBT:Cys molar ratios of R = 0.5, 1, 2, and 10. The chromotograph was shifted to the farthest left for R=1, indicating that an equimolar ratio of Cys:CBT is desirable to result in the largest crosslinked structures. A 2x fold molar excess of Cys (R=2) resulted in a much larger structure than a 2x fold molar excess of CBT (R=0.5), indicating that an excess of CKC relative to HA₁₀CBT is preferable for effective crosslinking *in situ.*

### Study 2. Impact of skin on HA-CBT crosslinking

HA₁₀CBT (1 mg/mL or 2 mg/mL) was incubated with human skin explant homogenate (50 mg skin/100 µL solution) at 37 °C. Crosslinker (CKC) was added at defined time points to the mixture to induce crosslinking. Following crosslinker addition, the samples were centrifuged to remove skin pieces, and the supernatant was run on GPC as previously described.

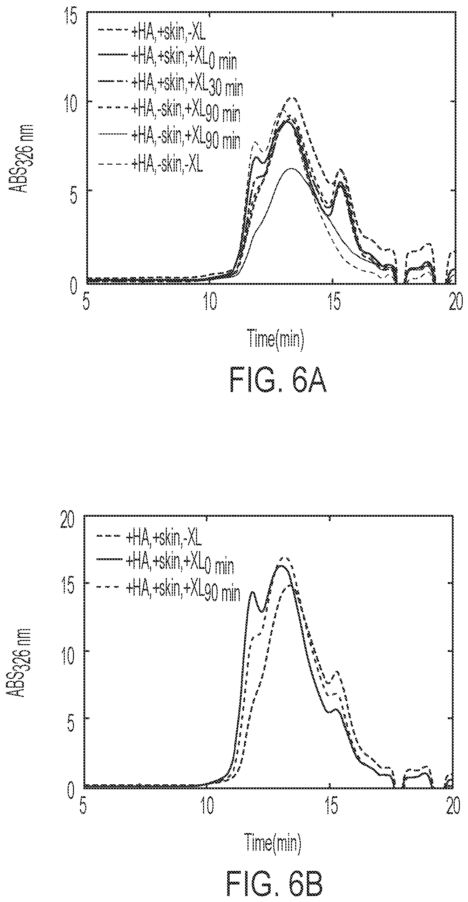

As shown in FIG. 6A, the spectrum of HA₁₀CBT after incubation with skin homogenate (+HA, +skin, -XL) increased in intensity with the same retention time compared to the pure HA₁₀CBT spectrum (+HA, -skin, -XL). This result may indicate a reaction with soluble aminothiols (e.g., free cysteine) in the homogenate solution occurred. When adding the crosslinker to the polymer/skin solution immediately after mixing the polymer with skin (+HA, +skin, +XL_{0 min}), the spectrum shifted to the left, indicating crosslinking occurred in the presence of the skin homogenate. However, when adding the crosslinker at 30 min (+HA, +skin, +XL_{30 min}), the shift was less noticeable indicating less crosslinking occurred. After 90 min incubation (+HA, +skin, +XL_{90 min}), the crosslinking was almost nonexistent as evident by the similarity in the spectrum to the spectrum from the sample with no crosslinker (+HA, +skin, -XL). On the contrary, crosslinking was still evident for the polymer that was incubated with skin homogenate for 90 min at a higher polymer concentration of 2 mg/mL as shown in FIG. 6B (+HA, +skin, +XL_{90 min}).

These results show that there is a finite time that HA₁₀CBT is active to react with the crosslinker CKC after incubating with the skin. However, if the polymer concentration is high enough (e.g., higher than 1 mg/mL HA-CBT in skin), then the polymer can remain active much longer. These results exemplify the importance of delivering HA₁₀CBT and the crosslinker within close temporal proximity to guarantee the two can efficiently crosslink *in situ.*

### Study 3. Impact of pH on HA-CBT crosslinking

To deliver HA-CBT and the crosslinker molecule topically through the skin, it is preferred that the two molecules do not crosslink until they reach to a target site. However, it is advantageous to deliver the two molecules simultaneously to increase the degree of crosslinking (as shown in FIG. 6) at the target site. The simultaneous delivery also allows for a single product application. To achieve simultaneous application without crosslinking exclusively on the skin surface, pH may be used to reduce the crosslinking reaction rate.

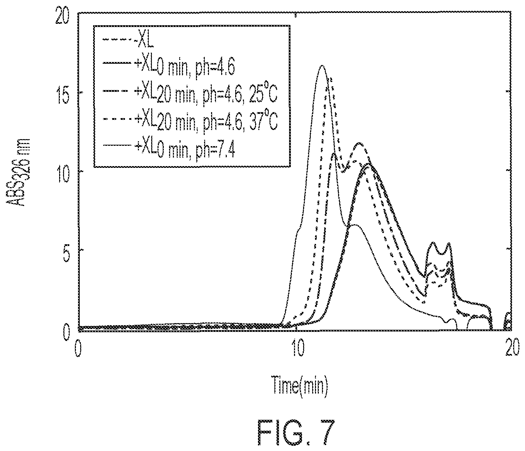

To demonstrate the impact of pH on reaction rate, HA₁₀CBT was mixed with the crosslinker molecule cysteine-ethylenediamine-cysteine at different pHs. Specifically, HA₁₀CBT (20 mg/mL) was mixed with CEC (at a Cys:CBT molar ratio = 1) at both neutral pH (PBS, pH = 7.4) and in an acidic buffer (25 mM citrate, pH = 4.6). As shown in FIG. 7, immediately after combining the two molecules, the two reacted in PBS forming a larger crosslinked structure (+XL_{0min, pH=7.4}). However, the GPC spectrum of the combined system at pH=4.6 (+XL_{0 min}, _{pH=4.6}) looked similar in molecular weight to the spectrum of the polymer alone (-XL), indicating that the two at pH = 4.6 did not react upon mixing. The combined system at pH = 4.6 gives time to apply the two products on the skin before crosslinking. Even after incubating the two components at 25 °C and 37 °C for 20 min, the final structure was still much less in molecular weight than the mixture that crosslinked immediately at neutral pH.

### Study 4. Crosslinking in situ in skin

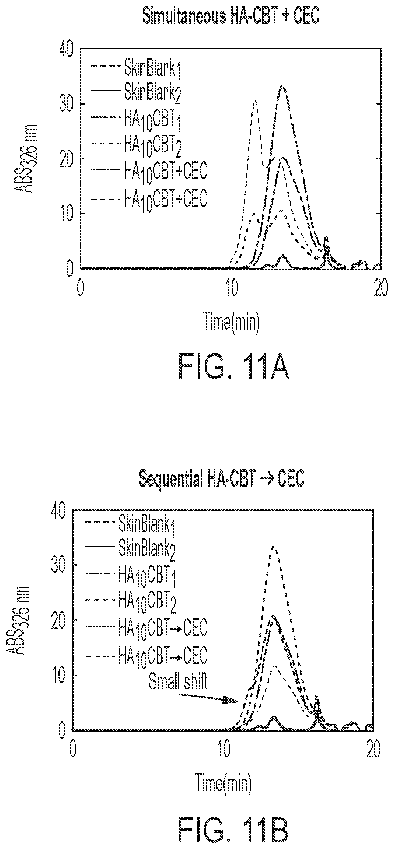

To demonstrate the ability to crosslink in the skin, HA₁₀CBT and CEC were injected in skin simultaneously and sequentially 30 minutes apart. As a control, HA₁₀CBT was also injected alone. As shown in FIG. 11A, there is a significant amount of crosslinked structure (shift to the left of HA₁₀CBT that is marked by the green arrow) when the two materials are injected at the same time. Less crosslinked structure is observed when there is a 30 minute delay between the injection of HA-CBT and CEC (FIG. 11B), verifying that delivery of the two materials in close temporal proximity results in better crosslinking.

### Example 3: Delivery of HA-CBT with microneedling and formation of in situ crosslinked structures (samples S5 and S6 are comparative examples and not according to the invention as claimed)

The present Example demonstrates impact of crosslinkable entity concentration on production and size of produced crosslinked material *in situ.* Among other things, the present Example defines particularly useful concentration(s) (e.g., of crosslinkable entities) and molar ratios *(e.g.,* of crosslink moieties and/or crosslinkable entities).

The present Example also documents impact of needle length on extent and/or size of crosslinked material formation *in situ.*

The procedure was similar to Example 1. Briefly, the surface of dermatomed human skin sections obtained from the abdomen of 40-50 yr. old female donors were cleaned with a solution of PBS and soap. The 2 cm² skin sections were then hydrated in warm PBS for about 30 minutes. Hydrated skin sections were then stretched and fixed with needles to keep it taut during microneedling with a dermaroller. Details pertaining to the concentration, volume and vehicle used for the polymer and crosslinker in each study are presented in Table 2. The final formulation to be tested was applied on the skin surface prior to microneedling. Dermarollers with needles of different lengths (250 µm, 500 µm, 1000 µm *etc.)* were tested for topical delivery and *in situ* crosslinking.

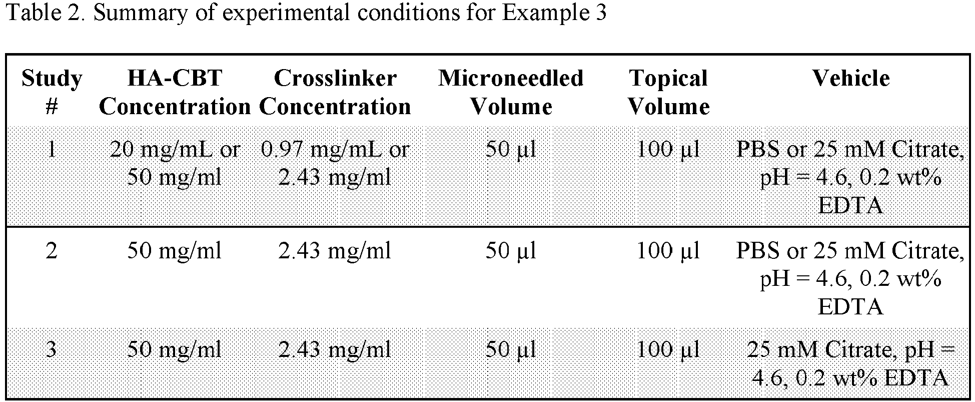

Following incubation, each skin piece was removed from the Franz diffusion cell and rinsed with PBS. The sections were then subjected to the tape-stripping method as previously described. The Upper Stratum Corneum (USC) and the Lower Stratum Corneum (LSC) were collected and placed in separate glass vials with 3 ml of PBS/DI water (1:1) mixture. The epidermis and the dermis were collected and placed in separate glass vials with 1 ml of PBS:DI water (1:1) mixture. The vials with the separated layers were then left to shake overnight at 37 °C to extract HA-CBT. The tissue extracts obtained were centrifuged to remove the tissue debris and the supernatants collected were concentrated via rotary evaporation and resuspended in 125 µl of DI water. The resuspended concentrates were centrifuged once again at 5000 g for 3 min and 90 µl of these purified supernatants were analyzed via GPC as previously described.

### Study 1. Impact of HA-CBT concentration on in situ crosslinking

HA-CBT formulations at 20 mg/mL and 50 mg/mL with an equimolar amount of the crosslinker CEC were applied topically in combination with microneedling. GPC chromatographs for the epidermal and dermal extracts for the higher concentration group (50 mg/mL) are shifted significantly to the left compared to the starting, un-crosslinked HA₁₀CBT. While the lower concentration group (20 mg/mL) also showed a GPC chromatograph shift for the epidermal extracts, the shift was much larger for the higher concentration group. The increase in HA-CBT concentration from 20 mg/mL to 50 mg/mL induced a qualitative change in the epidermal and dermal extract GPC spectrums, indicating that the crosslinking was much more effective for the 50 mg/mL group. Data from *ex vivo* crosslinking studies *(e.g.,* FIGS. 5A-5C), support the concept that there is a threshold concentration that may be achieved *in situ* to achieve effective crosslinking. This experiment suggests that an HA₁₀CBT concentration greater than 20 mg/mL may be topically applied in combination with microneedling (e.g., 250 µm dermaroller) to achieve effective crosslinking *in situ.*

### Study 2. Evidence of in situ crosslinking

HA₁₀CBT and the crosslinker cysteine-ethylenediamine-cysteine (CEC) were applied topically with microneedling in an un-crosslinked form and in a pre-crosslinked form. For the "un-crosslinked" formulation, HA₁₀CBT and CEC were mixed immediately prior to application and applied in an acidic buffer (25 mM citrate, pH=4.6, 0.2% EDTA). The un-crosslinked formulation shows significant reduction in the crosslinking reaction. For the "pre-crosslinked formulations", the two components were mixed 1h prior to application in the acidic buffer and in a neutral buffer (PBS). After application of the formulations with microneedling, the skin samples were further incubated in the Franz cells for 1 h at 37 °C, and then HA-CBT was extracted from each skin layer as previously described.

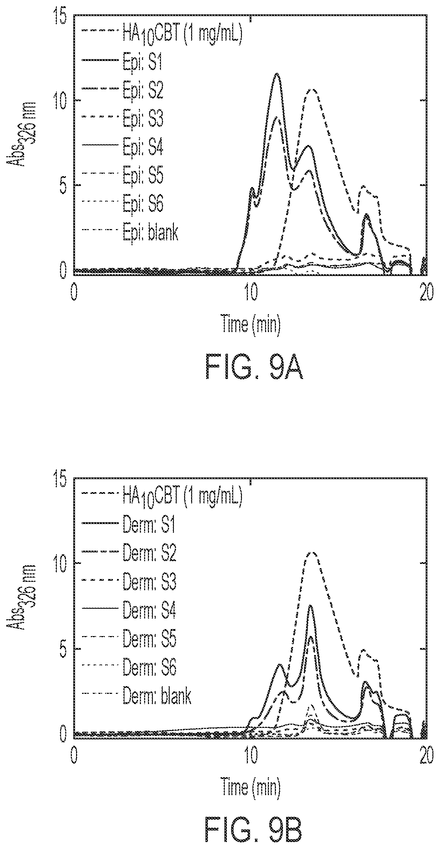

As shown in FIGS. 9A and 9B, extracts from skin sections that were microneedled with an un-crosslinked formulation revealed GPC spectra shifting to the left. The spectra indicates the formation of high molecular weight, crosslinked HA-CBT within the epidermis and dermis (S1, S2). However, the GPC spectrums obtained from extracts of skin sections that were treated with the pre-crosslinked formulations in acidic (S3 and S4), and neutral buffer (S5 and S6) were similar to the background tissue signal (blank). The absence of signals for the pre-crosslinked formulation highlights the advantage of using the un-crosslinked HA-CBT/Cys crosslinker system to build a network of higher molecular weight, crosslinked HA *in situ.* The low molecular weight HA variant (HA₁₀CBT) can penetrate the skin and then react and former larger structures *in situ,* as opposed to the pre-crosslinked formulations which are too large to penetrate the skin.

### Study 3. Impact of needle size

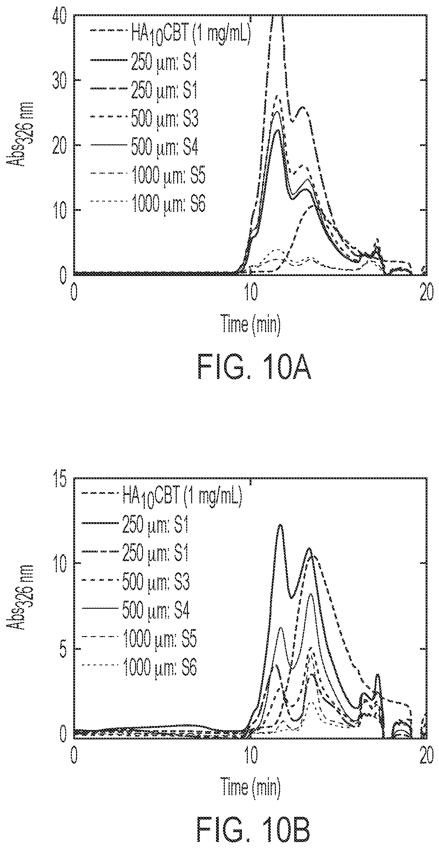

HA-CBT and CEC were delivered topically with microneedling using dermarollers of different needle lengths (250 µm, 500 µm, and 1000 µm). As shown in FIGS. 10A and 10B, extracts from skin sections revealed the GPC spectra shift farther left and increase in intensity with increasing needle size. Microneedling with a 500 µm (S3 and S4) and 1000 µm (S5 and S6) dermaroller delivered significantly more HA-CBT and formed larger *in situ* crosslinked structures (spectrum shifted farther left) in both the epidermis and dermis compared to microneedling with the smaller 250 µm dermaroller.

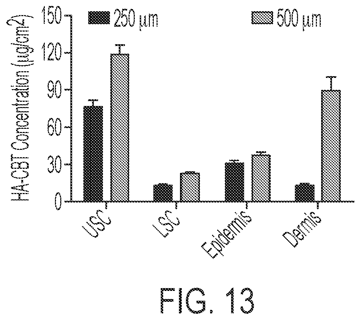

IR labeled material (HA₁₀CBT_{IR}) was also delivered identically with CEC using 250 µm and 500 µm dermarollers. A fluorescent plate reader (Tecan), as described elsewhere herein, was used to quantify the delivery of material. As shown in FIG. 13, significantly more HA-CBT is delivered into the epidermis and dermis using the larger dermaroller (500 µm), consistent with the GPC results.

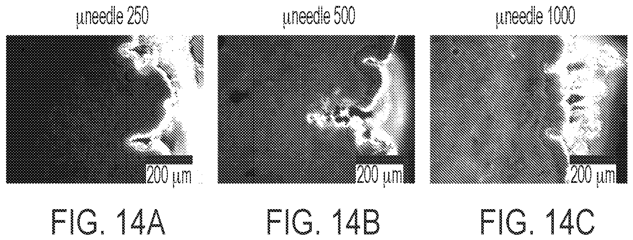

HA-CBT and CEC were delivered topically with microneedling using dermarollers of different needle lengths. Microscopy was also used to visualize the difference in delivery using dermarollers of different needle lengths. IR labeled material (HA₁₀CBT_{IR}) and CEC were delivered using the same application and incubation procedure as described immediately above. Following incubation, the skin sections were snap frozen in OCT and then sectioned on a cryostat microtome. Microscopy images were then taken on a Zeiss AxioPlan2 microscope using the 10x objective and Cy5 filter to visualize the location of the IR dye. As shown in FIG. 14A-C, much more fluorescence is observed in the dermis and epidermis for the treatment group with the larger microneedles.

### Example 4: in vivo study

The present Example describes the topical delivery of *in situ* crosslinked HA-CBT and the resulting persistence and safety. The present Example also demonstrates the impact of dermaroller size on HA-CBT delivery and persistence.

HA-CBT and the crosslinker (CEC) is administered (i) topically with a dermaroller or (ii) intradermally with injection in a mini pig model. Three Yorkshire mini-pigs, provided by CBSET - a contract research organization (CRO) (Lexinton, MA) - housed under standard environmental conditions with access to food and water is used. All animal experiments are reviewed and approved by the Institution Animal Care and Use Committee of CBSET.

The test groups for the proposed study are listed below. For GROUPS 4, 5 and 6, the test formulation are prepared by mixing equimolar solutions (CBT to Cys) of HA₁₀CBT and the crosslinker molecule cysteine-ethylenediamine-cysteine (CEC) in an acidic buffer (25 mM citrate buffer with 0.2% EDTA at pH 4.6) immediately prior to application. For GROUP 3 (Negative control 2), HA₁₀CBT is prepared in acidic buffer and applied without the crosslinker molecule, CEC. 50 µl of the formulation is applied. The dermaroller is rolled 10 times vertically to push the formulation into the skin. This is repeated 4 times in a vertical direction resulting in a total of 200 µl of the formulation applied in vertical direction to cover the entire skin area. Similarly, 50 µl of the formulation is applied at a direction right angle to the previous one. The dermaroller is rolled 10 times horizontally. This is repeated 4 times, thereby resulting in a total of 200 µl of the formulation applied in horizontal direction. Consequently, a total of 400 µl of the formulation is applied to the entire skin area to be treated.

### TEST Groups:

GROUP 1. Intradermally injected HA-CBT (50 mg/mL) with CEC (2.43 mg/ml) (Positive control)
GROUP 2. Topical use of Dermaroller 500 µm with PBS (Negative control 1)
GROUP 3. Topical use of Dermaroller 500 µm with HA-CBT (Negative control 2)
GROUP 4. Topical use of Dermaroller 250 µm for simultaneous delivery of HA-CBT (50 mg/ml) with the crosslinker molecule CEC (2.43 mg/ml) (Test group 1)
GROUP 5. Topical use of Dermaroller 500 µm for simultaneous delivery of HA-CBT (50 mg/ml) with the crosslinker molecule CEC (2.43 mg/ml) (Test group 2)
GROUP 6. Topical use of Dermaroller 1000 µm for simultaneous delivery of HA-CBT (50 mg/ml) with the crosslinker molecule CEC (2.43 mg/ml) (Test group 3)

Each animal has six sites (25-36 cm² per site) on each side of its back (Side A and Side B). On Day 0, the 6 different test groups are applied 1x at each site on Side A and Side B, for a total of 12 application regions per animal. The application sites are covered with a Tegaderm tape for an hour after which it will be removed.

On Day 1 following application, three (3) biopsies from each of the 6 "A" application regions (N=18 biopsies per pig) are taken and used for analyzing and measuring the amount of crosslinked HA-CBT in the skin. Analytical techniques (including UV plate reader and GPC) are used to quantify the amount of HA-CBT and characterize the size of HA-CBT in the skin. Microscopy is also used to visualize the location of HA-CBT within the skin, as CBT is fluorescent and HA-CBT is also labeled with an IR dye. The animals are then left without further applications for a 10-day period during which each site will be scored for irritation/redness, swelling/edema, flaking, cracking *etc.* Digital photographs are taken daily of the application sites.

On day 10, three (3) biopsies from each of the 6 "B" application regions (N=18 biopsies per pig) are obtained and used for analyzing and measuring the amount of HA-CBT that has persisted in the skin. Biopsies may also be fixed and sent for histological examination to understand the tolerability of the crosslinked HA-CBT within the pig skin (data not shown).

### Study 1. Delivery and in situ cross-linking of HA-CBT and CEC in vivo

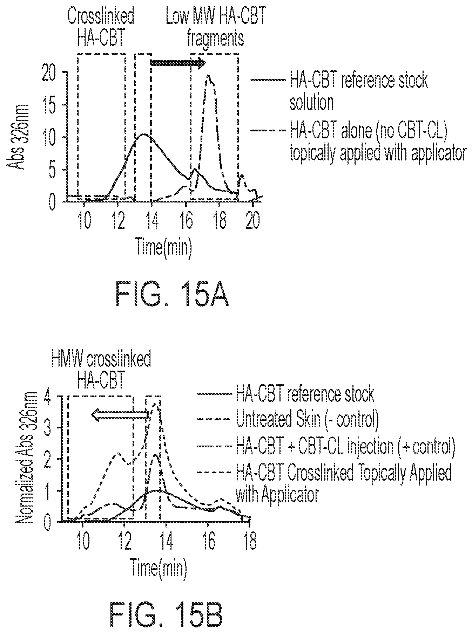

11 days following the application of HA-CBT and CEC, the pigs were euthanized. Skin samples were excised from each treatment group. A portion of the skin was fixed and saved for histology and another portion was used for GPC analysis (following the extraction with buffer, as described previously). FIG. 15A indicates that HA-CBT is detected in the skin 11 days after application when applied without CEC; however, there is a significant reduction in the molecular weight. In contrast, there is high molecular weight material detected in the skin 11 days following the application of HA-CBT and CEC (FIG. 15B). This indicates the material both crosslinked within the skin and persisted in a high molecular weight state in the skin for the duration of the study.

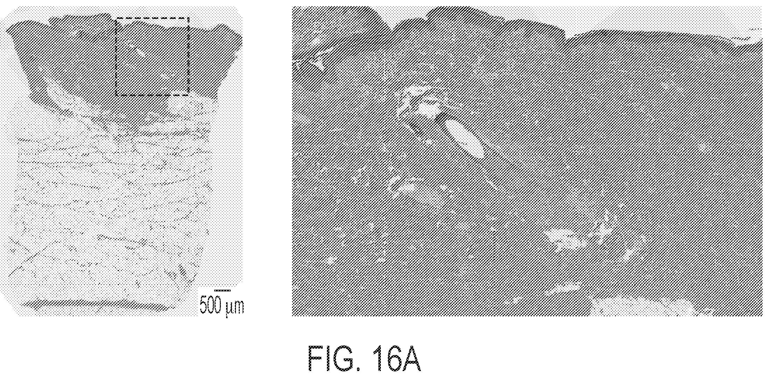

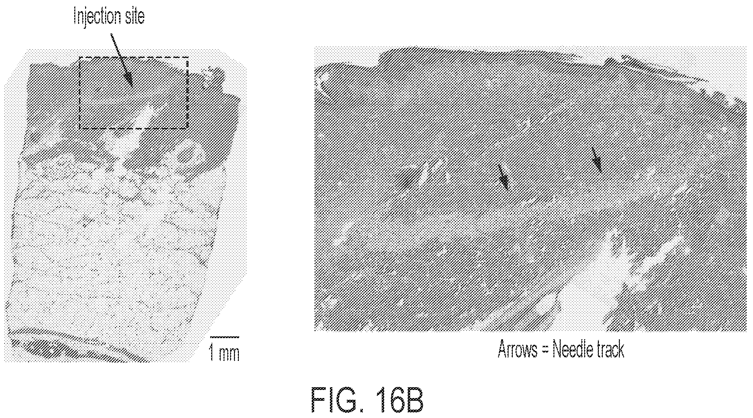

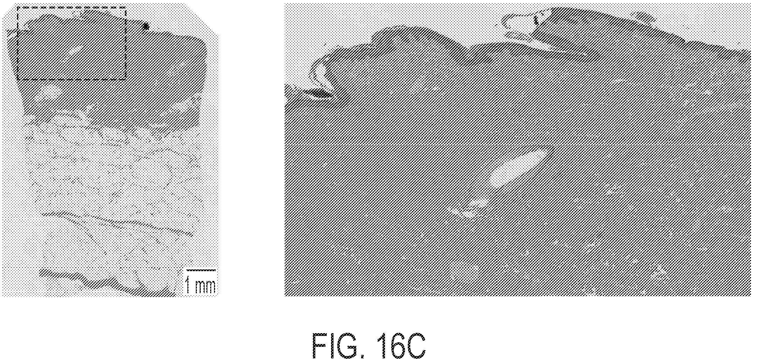

FIG. 16 shows the H & E stains for the skin 11 days after treatment with (A) Group 2, (B) Group 1, and (C) Group 5. Figure 16A shows that when buffer was dermarolled into the pig skin using a 500 µm derma-roller, no obvious side effects such as inflammation were observed 11 days after treatment. FIG. 16B further demonstrates that no inflammation is observed following the direct injection of HA-CBT and CEC into the skin. In addition, FIG. 16C demonstrates that no inflammation is observed following the application of HA-CBT and CEC with the dermaroller.

### Study 2. Evidence of HA in the superficial dermis

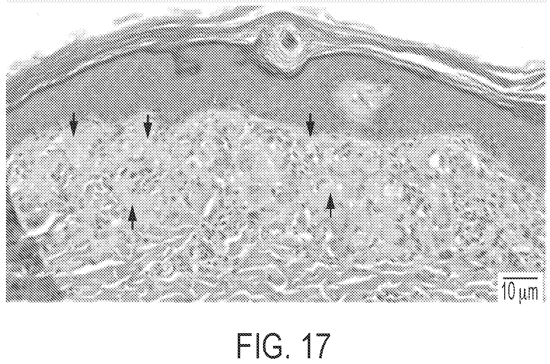

A follow-up study was conducted using a similar procedure as described in Study 1, but with repeat applications of formulations (on day 0 and day 14). FIG. 17 shows that when the crosslinked HA-CBT and CEC were dermarolled (using 500 µm dermarollers) into the pig skin twice (on day 0 and day 14), HA (shown in blue and pointed by arrows) were observed in the superficial dermis of the biopsy samples that were taken from the animals on day 28.

## Claims

1. A method of establishing a crosslinked material at an intradermal target site, the method comprising steps of:
(i) applying first and second crosslinkable entities to a skin location at a pH within a range of 3 ± 1% to 5 ± 1%; and
(ii) microneedling the skin location,
such that the crosslinked material becomes present at an intradermal target site, wherein: (a) the first crosslinkable entity comprises a crosslink moiety which is or comprises cyanobenzothiazole (CBT), a CBT mimetic, or another molecule that reacts with either the -SH or the NH2 group of cysteine; and (b) the second crosslinkable entity comprises a crosslink moiety which is or comprises cysteine (Cys), e.g., D-Cys, L-Cys, or combinations thereof.

2. The method of claim 1, wherein the microneedling is performed before and/or after the applying step.

3. The method of any preceding claims, wherein the presence of the crosslinked material is determinable at the intradermal target site.

4. The method of any preceding claims, wherein the crosslinked material becomes present within a time period of 1 minute to 1 hour.

5. The method of any preceding claims, wherein the first crosslinkable entity comprises a polymer moiety and a weight averaged molecular weight of the polymer moiety prior to the applying step is within a range of 1kDa to 500kDa, for example within a range of 5 to 20 kDa, optionally wherein the polymer moiety is hyaluronic acid ("HA") polymer.

6. The method of any preceding claims, wherein: the first and second crosslinkable entities are applied simultaneously, optionally further comprising mixing the first and second crosslinkable entities before the applying step, for example wherein the mixing step is performed 0 to 30 minutes before the applying step; or the second crosslinkable entity is applied after the first crosslinkable entity.

7. The method of any preceding claims, wherein the microneedling is performed with a microneedle device having microneedles, optionally wherein the microneedle device has a microneedle density within a range of 20 to 150 microneedles/cm² ± 25%, for example wherein: the microneedle device has 1 to 100000 microneedles; and/or the microneedles have a length between 100 µm ± 25% and 1000 µm ± 25%; and/or the microneedle device is a dermaroller.

8. The method of any preceding claims, wherein the intradermal site is epidermis (e.g., stratum corneum, stratum lucidum, stratum granulosum, stratum spinosum, stratum basale), or dermis.

9. The method of any preceding claims, wherein a concentration of the first crosslinkable entity is within a range of 0.1 to 100 mg/mL.

10. The method of any preceding claims, wherein: the second crosslinkable entity is selected from the group consisting of Cysteine-Ethylenediamine-Cysteine (CEC), Cysteine-Lysine-Cysteine (CKC), Cysteine-PEG-Cysteine, and combinations thereof; and/or comprises a crosslink moiety which is D-Cys, L-Cys, or combinations thereof.

11. The method of any preceding claims, wherein a concentration of the second crosslinkable entity is within a range of 0.1 to 100 mg/mL.

12. The method of any preceding claims, wherein a molecular ratio of the crosslink moiety of the first crosslinkable entity and the crosslink moiety of the second crosslinkable entity is within a range of 1:1 to 5:1.

13. The method of any preceding claims, wherein the first crosslinkable entity: comprises a crosslink moiety which is CBT or a CBT mimetic; and/or comprises a crosslink moiety, and 1-20 mol % of the first crosslinkable entity comprises the crosslink moiety.

14. The method of any preceding claims, wherein the crosslinked material is **characterized in that** a weight averaged molecular weight of the crosslinked material at the intradermal target site is greater than a weight averaged molecular weight of the first crosslinkable entity, for example two, three, four, five, six, seven, eight, nine or ten times greater.

## Patentansprüche

1. Verfahren zum Ausbilden eines vernetzten Materials an einem intradermalen Zielort, das Verfahren umfassend die Folgenden Schritte:
(i) Aufbringen einer ersten und einer zweiten vernetzbaren Einheit auf eine Hautstelle bei einem pH-Wert innerhalb eines Bereichs von 3 ± 1 % bis 5 ± 1 %; und
(ii) Microneedling der Hautstelle,
derart, dass das vernetzte Material an einem intradermalen Zielort präsent wird, wobei: (a) die erste vernetzbare Einheit einen Vernetzungsanteil umfasst, der Cyanobenzothiazol (CBT), ein CBT-Mimetikum oder ein anderes Molekül ist oder umfasst, das mit entweder der -SH- oder der NH2-Gruppe von Cystein reagiert; und (b) die zweite vernetzbare Einheit einen Vernetzungsanteil umfasst, der Cystein (Cys), z. B. D-Cys, L-Cys oder Kombinationen davon, ist oder umfasst.

2. Verfahren nach Anspruch 1, wobei das Microneedling vor und/oder nach dem Schritt des Aufbringens durchgeführt wird.

3. Verfahren nach einem der vorstehenden Ansprüche, wobei die Präsenz des vernetzten Materials an dem intradermalen Zielort bestimmbar ist.

4. Verfahren nach einem der vorstehenden Ansprüche, wobei das vernetzte Material innerhalb einer Zeitspanne von 1 Minute bis 1 Stunde präsent wird.

5. Verfahren nach einem der vorstehenden Ansprüche, wobei die erste vernetzbare Einheit einen Polymeranteil umfasst und ein gewichtsgemitteltes Molekulargewicht des Polymeranteils vor dem Schritt des Aufbringens innerhalb eines Bereichs von 1 kDa bis 500 kDa liegt, zum Beispiel innerhalb eines Bereichs von 5 bis 20 kDa, wahlweise wobei der Polymeranteil ein Hyaluronsäurepolymer ("HA"-Polymer) ist.

6. Verfahren nach einem der vorstehenden Ansprüche, wobei: die erste und die zweite vernetzbare Einheit gleichzeitig aufgebracht werden, wahlweise ferner umfassend ein Mischen der ersten und der zweiten vernetzbaren Einheit vor dem Schritt des Aufbringens, zum Beispiel wobei der Schritt des Mischens 0 bis 30 Minuten vor dem Schritt des Aufbringens durchgeführt wird; oder die zweite vernetzbare Einheit nach der ersten vernetzbaren Einheit aufgebracht wird.

7. Verfahren nach einem der vorstehenden Ansprüche, wobei das Microneedling mit einer Mikronadelvorrichtung, die Mikronadeln aufweist, durchgeführt wird, wahlweise wobei die Mikronadelvorrichtung eine Mikronadeldichte innerhalb eines Bereichs von 20 bis 150 Mikronadeln/cm² ± 25 % aufweist, zum Beispiel wobei: die Mikronadelvorrichtung 1 bis 100000 Mikronadeln aufweist; und/oder die Mikronadeln eine Länge zwischen 100 µm ± 25 % und 1000 µm ± 25 % aufweisen; und/oder die Mikronadelvorrichtung ein Dermaroller ist.

8. Verfahren nach einem der vorstehenden Ansprüche, wobei der intradermale Ort die Epidermis (z.B. Stratum corneum, Stratum lucidum, Stratum granulosum, Stratum spinosum, Stratum basale) oder die Dermis ist.

9. Verfahren nach einem der vorstehenden Ansprüche, wobei eine Konzentration der ersten vernetzbaren Einheit innerhalb eines Bereichs von 0,1 bis 100 mg/ml liegt.

10. Verfahren nach einem der vorstehenden Ansprüche, wobei: die zweite vernetzbare Einheit aus der Gruppe bestehend aus Cysteinethylendiamincystein (CEC), Cysteinlysincystein (CKC), Cystein-PEG-cystein und Kombinationen davon ausgewählt ist; und/oder einen Vernetzungsanteil umfasst, der D-Cys, L-Cys oder Kombinationen davon ist.

11. Verfahren nach einem der vorstehenden Ansprüche, wobei eine Konzentration der zweiten vernetzbaren Einheit innerhalb eines Bereichs von 0,1 bis 100 mg/ml liegt.

12. Verfahren nach einem der vorstehenden Ansprüche, wobei ein Molekularverhältnis des Vernetzungsanteils der ersten vernetzbaren Einheit und des Vernetzungsanteils der zweiten vernetzbaren Einheit innerhalb eines Bereichs von 1 : 1 bis 5 : 1 liegt.

13. Verfahren nach einem der vorstehenden Ansprüche, wobei die erste vernetzbare Einheit: einen Vernetzungsanteil umfasst, der CBT oder ein CBT-Mimetikum ist; und/oder einen Vernetzungsanteil umfasst und 1-20 Mol-% der ersten vernetzbaren Einheit den Vernetzungsanteil umfasst.

14. Verfahren nach einem der vorstehenden Ansprüche, wobei das vernetzte Material **dadurch gekennzeichnet ist, dass** ein gewichtsgemitteltes Molekulargewicht des vernetzten Materials an dem intradermalen Zielort größer als ein gewichtsgemitteltes Molekulargewicht der ersten vernetzbaren Einheit, zum Beispiel zwei-, drei-, vier-, fünf-, sechs-, sieben-, acht-, neun- oder zehnmal größer, ist.

## Revendications

1. Procédé d'établissement d'un matériau réticulé au niveau d'un site cible intradermique, le procédé comprenant les étapes consistant à :
(i) l'application de première et seconde entités réticulables à un emplacement de peau à un pH compris dans une plage de 3 ± 1 % à 5 ± 1 % ; et
(ii) l'utilisation de micro-aiguilles à l'emplacement de peau,
de telle sorte que le matériau réticulé devient présent au niveau d'un site cible intradermique, dans lequel : (a) la première entité réticulable comprend un fragment de réticulation qui est ou comprend du cyanobenzothiazole (CBT), un mimétique de CBT, ou une autre molécule qui réagit avec soit le groupe -SH soit le groupe NH2 de la cystéine ; et (b) la seconde entité réticulable comprend un fragment de réticulation qui est ou comprend de la cystéine (Cys), par ex., D-Cys, L-Cys, ou des combinaisons de celles-ci.

2. Procédé selon la revendication 1, dans lequel l'utilisation de micro-aiguilles est effectuée avant et/ou après l'étape d'application.

3. Procédé selon l'une quelconque des revendications précédentes, dans lequel la présence du matériau réticulé peut être déterminée au niveau du site cible intradermique.

4. Procédé selon l'une quelconque des revendications précédentes, dans lequel le matériau réticulé devient présent dans un laps de temps de 1 minute à 1 heure.

5. Procédé selon l'une quelconque des revendications précédentes, dans lequel la première entité réticulable comprend un fragment polymère et une masse moléculaire moyennée en poids du fragment polymère avant l'étape d'application est dans une plage de 1 kDa à 500 kDa, par exemple dans une plage de 5 à 20 kDa, facultativement dans lequel le fragment polymère est un polymère d'acide hyaluronique («HA»).

6. Procédé selon l'une quelconque des revendications précédentes, dans lequel : les première et seconde entités réticulables sont appliquées simultanément, comprenant facultativement en outre le mélange des première et seconde entités réticulables avant l'étape d'application, par exemple dans lequel l'étape de mélange est effectuée 0 à 30 minutes avant l'étape d'application ; ou la seconde entité réticulable est appliquée après la première entité réticulable.

7. Procédé selon l'une quelconque des revendications précédentes, dans lequel l'utilisation de micro-aiguilles est effectuée avec un dispositif à micro-aiguilles ayant des micro-aiguilles, éventuellement dans lequel le dispositif à micro-aiguilles a une densité de micro-aiguilles dans une plage comprise entre 20 et 150 micro-aiguilles/cm² ± 25 %, par exemple dans lequel : le dispositif à micro-aiguilles a 1 à 100 000 micro-aiguilles ; et/ou les micro-aiguilles ont une longueur comprise entre 100 µm ± 25 % et 1 000 µm ± 25 % ; et/ou le dispositif à micro-aiguille est un dermaroller.

8. Procédé selon l'une quelconque des revendications précédentes, dans lequel le site intradermique est un épiderme *(par ex.,* stratum corneum, stratum lucidum, stratum granulosum, stratum spinosum, stratum basale), ou un derme.

9. Procédé selon l'une quelconque des revendications précédentes, dans lequel une concentration de la première entité réticulable est comprise dans une plage de 0,1 à 100 mg/mL.

10. Procédé selon l'une quelconque des revendications précédentes, dans lequel : la seconde entité réticulable est choisie dans le groupe constitué de cystéine-éthylènediamine-cystéine (CEC), cystéine-lysine-cystéine (CKC), cystéine-PEG-cystéine, et des combinaisons de ceux-ci ; et/ou comprend un fragment de réticulation qui est D-Cys, L-Cys, ou des combinaisons de ceux-ci.

11. Procédé selon l'une quelconque des revendications précédentes, dans lequel une concentration de la seconde entité réticulable est comprise entre 0,1 et 100 mg/mL.

12. Procédé selon l'une quelconque des revendications précédentes, dans lequel un rapport moléculaire de la fraction de réticulation de la première entité réticulable et de la fraction de réticulation de la seconde entité réticulable est dans une plage de 1:1 à 5:1.

13. Procédé selon l'une quelconque des revendications précédentes, dans lequel la première entité réticulable : comprend un fragment de réticulation qui est une TCC ou un mimétique de TCC ; et/ou comprend un fragment de réticulation, et 1 à 20 % molaires de la première entité réticulable comprend le fragment de réticulation.

14. Procédé selon l'une quelconque des revendications précédentes, dans lequel le matériau réticulé est **caractérisé en ce qu'**une masse moléculaire moyenne en poids du matériau réticulé au niveau du site cible intradermique est supérieure à une masse moléculaire moyenne en poids de la première entité réticulable, par exemple deux, trois, quatre, cinq, six, sept, huit, neuf ou dix fois plus grande.
